# FASCICULE DE BREVET EUROPEEN

(11) **EP 3 922 628 B1**
(45) Date de publication et mention de la délivrance du brevet: **19.02.2025**
(21) Numéro de dépôt: 21185886.5
(22) Date de dépôt: 09.04.2015
(51) Int. Cl.: C07D 215/48, C07D 215/14, C07D 215/18, A61K 31/4184, A61P 37/00

(54) **COMPOSÉS CYTOTOXIQUES INHIBITEURS DE LA POLYMÉRISATION DE LA TUBULINE**
ZYTOTOXISCHE VERBINDUNGEN ALS INHIBITOREN DER POLYMERISATION VON TUBULIN
CYTOTOXIC COMPOUNDS INHIBITING TUBULIN POLYMERISATION

(30) Priorité: 09.04.2014 FR 1453142
(43) Date de publication de la demande: 15.12.2021
(62) Demande divisionnaire de: 15716014.4
(73) Titulaire: Université Paris-Saclay, 91190 Gif-sur-Yvette (FR); Centre national de la recherche scientifique, 75016 Paris (FR)
(72) Inventeur: ALAMI, Mouad, 77660 BUSSY SAINT GEORGES (FR); BRION, Jean-Daniel, 95320 SAINT LEU LA FORET (FR); MESSAOUDI, Samir, 91380 CHILLY MAZARIN (FR); PROVOT, Olivier, 78500 SARTROUVILLE (FR); SOUSSI, Mohamed-Ali, 2037 ARIAN (TN); BIGNON, Jérôme, 91530 LA VAL SAINT GERMAIN (FR); DUBOIS, Joëlle, 91400 GOMETZ-LA-VILLE (FR); BAKALA-WDZIECZAK, Joanna, 75012 PARIS (FR)
(74) Mandataire: Novagraaf Technologies

(56) Documents cités:
- WO-A1-2008/122620
- US-A- 2 616 890
- US-A- 5 041 453
- LAWSON MARIE ET AL: "An efficient coupling of N-tosylhydrazones with 2-halopyridines: synthesis of 2-[alpha]-styrylpyridines endowed with antitumor activity", ORGANIC & BIOMOLECULAR CHEMISTRY, vol. 11, no. 22, 1 January 2013 (2013-01-01), pages 3664, XP055849753, ISSN: 1477-0520, Retrieved from the Internet <URL:https://pubs.rsc.org/en/content/articlepdf/2013/ob/c3ob40263k> DOI: 10.1039/c3ob40263k
- LEONARD ET AL: "Exploring molecular shape analysis of styrylquinoline derivatives as HIV-1 integrase inhibitors", EUROPEAN JOURNAL OF MEDICINAL CHEMISTRY, ELSEVIER, AMSTERDAM, NL, vol. 43, no. 1, 1 January 2008 (2008-01-01), pages 81 - 92, XP022423016, ISSN: 0223-5234, DOI: 10.1016/J.EJMECH.2007.02.021
- LI YUNFENG ET AL: "Iron-Catalyzed Synthesis of 2-Vinylquinolines via sp 3 C-H Functionalization and Subsequent C-N Cleavage", CHEMISTRY - AN ASIAN JOURNAL, vol. 8, no. 3, 23 January 2013 (2013-01-23), pages 534 - 537, XP055850117, ISSN: 1861-4728, DOI: 10.1002/asia.201201039

## Description

### DOMAINE DE L'INVENTION

La présente invention concerne des composés inhibiteurs de la polymérisation de la tubuline, leurs procédés de préparation ainsi que leurs utilisations.

### ETAT DE LA TECHNIQUE

Le cancer est une cause majeure de décès dans le monde, à l'origine de 7,6 millions de décès en 2008, soit environ 13% de la mortalité mondiale. D'après les estimations de l'OMS, la mortalité due au cancer va continuer à augmenter pour dépasser les 13 millions de décès en 2030. Environ 30% des décès par cancer sont dus à cinq principaux facteurs de risque comportementaux et alimentaires: (*i*) un indice élevé de masse corporelle, (*ii*) une faible consommation de fruits et de légumes, (*iii*) le manque d'exercice physique, (*iv*) le tabagisme et (*v*) la consommation d'alcool. Le cancer peut être réduit et endigué en appliquant des stratégies fondées sur, des bases factuelles pour la prévention, le dépistage précoce, la prise en charge des patients et les traitements spécifiques. Concernant ce dernier point, il existe trois modalités principales de traitement des cancers: la chirurgie, la radiothérapie et la chimiothérapie. Pour certains types de cancers, il est également possible de faire appel à l'hormonothérapie, à l'immunothérapie ou aux récentes thérapies ciblées. Selon les cas, ces modalités peuvent être prescrites seules, successivement ou en association. La chimiothérapie ou les polychimiothérapies consistent à utiliser plusieurs composés cytotoxiques pour la cellule cancéreuse. À la différence de la chirurgie, il s'agit de traitements systémiques qui concernent l'ensemble du corps et non un organe particulier et qui auront cependant des conséquences sur les cellules saines comme des effets secondaires plus ou moins marqués.

Une des caractéristiques de la cellule cancéreuse est de se diviser très rapidement pour permettre à la tumeur de se développer. Cette particularité est « ciblée » par divers agents qui vont perturber la division cellulaire comme par exemple, les intercalants de l'ADN (daunorubicine, doxorubicine, mitoxandrone,...), les inhibiteurs de topoisomérases I et II qui modifient la structure tertiaire de l'ADN (irinotecan, l'étoposide,...) ou bien encore les poisons du fuseau mitotique.

L'importance des microtubules en tant que cibles anticancéreuses est soulignée par l'utilisation clinique de plusieurs agents ciblant les microtubules dans le traitement des cancers, comme les alcaloïdes de la pervenche de Madagascar de type Vinca et les taxanes. Les alcaloïdes de la pervenche (vincristine, vinblastine,...) établissent une liaison spécifique à la tubuline et inhibent sa polymérisation en microtubules empêchant ainsi la formation du fuseau mitotique. Les taxanes (paclitaxel, docétaxel) empêchent quant à eux la dépolymérisation de la tubuline et perturbent également l'équilibre tubuline-microtubules. Ainsi, le désassemblage du fuseau mitotique est bloqué ce qui provoque, l'arrêt de la mitose entraînant la mort de la cellule. Le docétaxel est préconisé dans le traitement de très nombreux cancers. Il est utilisé seul ou en association avec un anti-inflamatoire (prednisone,...) dans le cancer avancé de la prostate (premier cancer de l'homme en France). Dans les cas de cancers du sein (premier cancer de la femme), le docétaxel s'utilise seul ou en association avec d'autres agents antitumoraux (doxorubicine, trastuzmab,...). Dans le cancer du poumon non à petites cellules (second cancer de l'homme et troisième de la femme) il a montré son efficacité seul ou associé au cis-platine. Enfin, le docétaxel, en association avec le 5-fluorouracile, est donné pour traiter les cas de cancer de l'estomac, de la tête et du cou. Le taxol a trouvé son application dans les traitements de première intention, dans le cancer du sein associé à une anthracycline et dans le cancer de l'ovaire associé au cis-platine.

Malgré l'efficacité en thérapeutique de ces traitements, ces divers composés sont à l'origine de nombreux effets secondaires (alopécie, neutropénie, nausées, mucites, douleurs musculaires,...). Par exemple les alcaloïdes de la pervenche ont été associés à des problèmes de neuro-, hémato- et cardiotoxicité. Par ailleurs, des phénomènes de résistance dus à des changements phénotypiques des cellules cancéreuses sont de plus en plus observés, comme par exemple dans le cas des cancers de la prostate non hormono-dépendants traités par le docétaxel.

Des recherches ont été entreprises pour identifier et développer de nouvelles molécules perturbant l'assemblage de la tubuline en microtubules.

Dans ce cadre, la combrétastatine A-4 (CA-4), appartenant à la famille des combrétastatines, stilbènes naturels isolés par G. R. Pettit de l'écorce d'un saule sud-africain, *Combretum caffrum*¹*,* a été identifiée. La CA-4 est un stilbène de configuration Z substitué par deux noyaux aromatiques de type 3,4,5-triméthoxyphényle (cycle A) et 3-hydroxy-4-méthoxyphényle (cycle B). La CA-4 s'est révélée être très cytotoxique (Cl₅₀ = 1-2 nM) vis à vis de nombreuses lignées cancéreuses humaines ainsi que vis à vis de lignées résistantes aux thérapies conventionnelles utilisant par exemple, la daunorubicine². La CA-4 est également un poison du fuseau mitotique puisqu'elle inhibe fortement l'assemblage de la tubuline en microtubules en se fixant au site de la colchicine (Cl₅₀ = 1 µM). Il a également été démontré que la CA-4 présente une activité antivasculaire³ nanomolaire *in vitro* par inhibition de la prolifération de cellules endothéliales. Une prodrogue hydrosoluble de la CA-4, la fosbrétabuline (CA-4P), est actuellement en développement clinique de phase III dans le traitement du cancer de la thyroïde et en développement clinique de phase II dans les traitements du cancer des poumons non à petites cellules et dans les traitements des cancers ovariens résistants au cis-platine. De même, le chlorhydrate d'un analogue structural de la CA-4, l'ombrabuline, est actuellement utilisé pour traiter les sarcomes avancés des tissus mous.

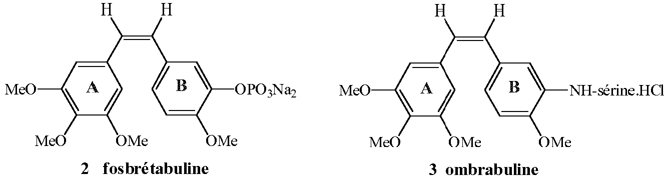

Si ces composés s'avèrent efficaces, ils présentent une instabilité chimique imputable à l'isomérisation de la double liaison éthylénique.

L'*iso*combrétastatine A-4 (CA-4),⁴ isomère non naturel de la CA-4, dont le profil biologique (cytotoxicité, inhibition de la polymérisation de la tubuline, induction de l'apoptose,...) est rigoureusement identique à celui de la molécule naturelle, sans toutefois présenter le risque d'isomérisation a été récemment identifié. Cette molécule, particulièrement stable, ne se métabolise que très peu (<10%) en présence d'hépatocytes.⁵

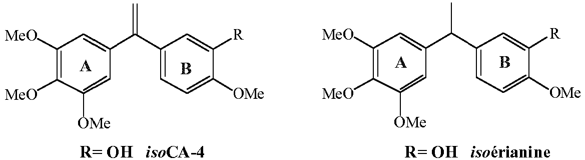

Il a été également démontré qu'il est possible de réduire la double liaison de l'isoCA-4 sans perte notoire d'activité antitumorale.⁶ Ainsi, l'isoérianine, isomère non naturel de l'érianine, s'est révélée cytotoxique à des concentrations nanomolaires (25<GI₅₀<45 nM) sur de nombreuses lignées tumorales humaines et inhibe la polymérisation de la tubuline à des concentrations micromolaire en bloquant le cycle cellulaire en phase G2/M.

La demande WO 2008/122620 divulgue des inhibiteurs de la polymérisation de la tubuline utiles pour le traitement du cancer et de structure analogue à l'isocombrétastatine A-4.

Les analogues de la combréstatine A-4 synthétisés à ce jour présentent un motif 3,4,5-triméthophényl. Ce groupement étant suspecté d'être à l'origine d'effets secondaires, tels qu'une neurotoxicité ou cardiotoxicité, un besoin existe pour la mise à disposition de nouveaux agents inhibiteurs de la polymérisation de la tubuline. Idéalement, ces composés devront: (i) être efficaces à des doses peu ou pas toxiques pour l'homme, (*ii*) être stables et facile à produire industriellement, (*iii*) être solubles dans l'eau pour simplifier leur mode d'administration, (*iv*) avoir un mécanisme d'action identifié et, (*v*) être dépourvus si possible d'effets secondaires.

### BREVE DESCRIPTION DE L'INVENTION

La présente invention est uniquement définie par les revendications. Elle porte sur un composé de formule (Ib) tel que définie dans les revendications. Ladite formule (Ib) est liée à la formule (I) suivante : 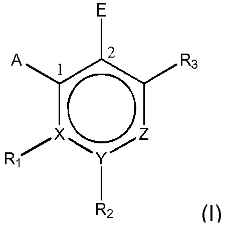 dans laquelle :
- R₂ et R₃ sont différents et un des deux représente un groupement A₁ de formule suivante : 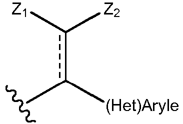 dans lequel :
   - la liaison en pointillé est présente ou absente, de préférence présente ;
   - Z₁ et Z₂ représentent indépendamment l'un de l'autre un atome d'hydrogène, un atome d'halogène ou un groupe méthyle ;
   - (Het)Aryle représente un groupe aryle ou hétéroaryle, ledit hétéroaryle étant choisi parmi les groupes indolyle, benzothiophényle et benzofuranyle, ledit aryle ou hétéroaryle pouvant être substitué par un ou plusieurs substituants choisis parmi un atome d'halogène, un groupe hydroxyle, -OMe, -SMe, - OCX₃ avec X désignant un atome d'halogène, -NH₂, 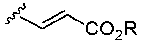 avec R désignant un groupe alkyle en C₁ à C₆, 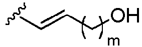 avec m représentant 1 ou 2, 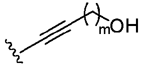 avec m représentant 1 ou 2, et

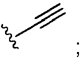

   l'autre représentant alors
   un atome d'hydrogène,
   un atome d'halogène,
   un groupe hydroxyle,
   un groupe cyano,
   un groupe -COYR' avec Y désignant O ou N et R' désignant H ou un groupe alkyle en C₁ à C₆, un groupe alcényle en C₂ à C₄, un groupe alcynyle en C₂ à C₄,
   un groupe -SO₂NR'R" avec R', R", désignant chacun, indépendamment l'un de l'autre, H ou un groupe alkyle en C₁ à C₆, un groupe alcényle en C₂ à C₄, un groupe alcynyle en C₂ à C₄, un groupe aryle, un groupe hétéroaryle
   un groupe -NHSO₂R' avec R' désignant un groupe alkyle en C₁ à C₆, un groupe alcényle en C₂ à C₄, un groupe alcynyle en C₂ à C₄, un groupe aryle, un groupe hétéroaryle
   un groupe alkyle en C₁ à C₆,
   un groupe alcényle en C₂ à C₄,
   un groupe alcynyle en C₂ à C₄,
   un groupe alkoxy en C₁ à C₆
   ou un groupe -NR'R" avec R' et R" représentant, indépendamment l'un de l'autre un hydrogène ou un groupe alkyle ;
- A et E représentent chacun un atome d'hydrogène ou A et E participent à la structure d'un cycle aromatique accolé de formule suivante : 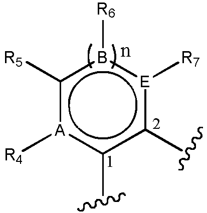 dans laquelle A, B et E représentent, indépendamment l'un de l'autre, un atome de carbone ou d'azote, n représente 0 ou 1 et R₄, R₅, R₆, R₇ sont tels que décrits ci-dessous ;
- X, Y et Z représentent, indépendamment l'un de l'autre, un atome de carbone ou d'azote à la condition que si X et Z représentent un atome d'azote, Y représente un atome de carbone ;
- au moins un de A, B, E, X, Y et Z représente un atome d'azote ;
- R₁, R₄, R₅, R₆, R₇, si présents, représentent, indépendamment l'un de l'autre un atome d'hydrogène, un atome d'halogène, un groupe hydroxyle, un groupe alkyle en C₁ à C₆, un groupe alcényle en C₂ à C₄, un groupe alcynyle en C₂ à C₄, un groupe alkoxy en C₁ à C₆ ou un groupe -NR'R" avec R' et R" représentant, indépendamment l'un de l'autre un hydrogène ou un groupe alkyle en C₁ à C₆ ;
ainsi que ses sels pharmaceutiquement acceptables, ses stéréoisomères et ses prodrogues.

La présente invention porte également sur une composition pharmaceutique comprenant au moins un composé de la présente invention.

La présente invention porte aussi sur les composés ou compositions de la présente invention pour leur utilisation en tant que médicament, en particulier destiné à traiter ou prévenir les maladies prolifératives ou médicament anti-vasculaire.

### DEFINITIONS

Le terme "halogène", tel qu'utilisé dans la description de la présente invention, désigne les atomes de fluor, chlore, brome et iode. De manière avantageuse, il s'agira du fluor, du brome et du chlore et encore plus avantageusement du fluor ou du chlore.

Le terme "alkyle en C₁ à C₆", tel qu'utilisé dans la description de la présente invention, désigne tout groupe hydrocarboné saturé comportant de 1 à 6 atomes de carbone, linéaire ou ramifié, en particulier les groupes méthyle, éthyle, *n*-propyle, isopropyle, *n*-butyle, *iso*-butyle, *sec*-butyle, *tert*-butyle, pentyle et hexyle.

Le terme "alcényle en C₂ à C₄", tel qu'utilisé dans la description de la présente invention, désigne tout groupe hydrocarboné comportant de 2 à 4 atomes de carbone, linéaire ou ramifié, et comportant au moins une double liaison, tel qu'un groupe vinyle (éthényle).

Le terme "alcynyle en C₂ à C₄", tel qu'utilisé dans la description de la présente invention, désigne tout groupe hydrocarboné comportant de 2 à 4 atomes de carbone, linéaire ou ramifié, et comportant au moins une triple liaison, tel qu'un groupe éthynyle ou propynyle.

Le terme "alkoxy en C₁ à C₆", tel qu'utilisé dans la description de la présente invention, désigne tout groupe -O-alkyle, l'alkyle étant tel que défini ci-dessus. Des exemples de groupes alkoxy incluent les groupes méthoxy, éthoxy, propoxy, *n*-butoxy, *iso*-butoxy et *tert*-butoxy.

Le terme "(het)Aryle", tel qu'utilisé dans la description de la présente invention, désigne un aryle ou hétéroaryle.

Le terme " aryle", tel qu'utilisé dans la description de la présente invention, désigne un ou plusieurs cycles aromatiques ayant de 5 à 10 atomes de carbone, pouvant être accolés. En particulier, les groupes aryles peuvent être des groupes monocycliques ou bicycliques, comme par exemple le groupe phényle ou naphthyle. Avantageusement, le groupe aryle est un phényle.

Le terme "hétéroaryle", tel qu'utilisé dans la description de la présente invention, désigne un groupe aromatique comprenant de 5 à 10 atomes cycliques. Les atomes cycliques comprennent des atomes de carbone et un ou plusieurs hétéroatomes, tels que par exemple des atomes de soufre, d'azote ou d'oxygène. L'hétéroaryle selon la présente invention peut être constitué par un ou deux cycles accolés. De préférence, le groupe hétéroaryle sera un groupe indolyle, benzothiophényle, benzofuranyle ou benzoimidazolyle.

Le terme « cyano », tel qu'utilisé dans la description de la présente invention, désigne un groupe -CN.

La formule « -COYR' », telle qu'utilisée dans la description de la présente invention, désigne un acide ou un ester lorsque Y est O, un amide lorsque Y est N.

La formule « -SO₂NR'R" » ou « - NHSO₂R' », telle qu'utilisée dans la description de la présente invention, désigne un sulfonamide.

L'expression "pharmaceutiquement acceptable", telle qu'utilisée dans la description de la présente invention, désigne ce qui est utile dans la préparation d'une composition pharmaceutique, qui est généralement sûr, non toxique et ni biologiquement ni autrement non souhaitable et qui est acceptable pour une utilisation vétérinaire et/ou pharmaceutique humaine.

L'expression "sels pharmaceutiquement acceptables", telle qu'utilisée dans la description de la présente invention, désigne des sels d'un composé qui sont pharmaceutiquement acceptables, comme définis ici, et qui possèdent l'activité pharmacologique souhaitée du composé parent. De tels sels comprennent :
(1) les hydrates et les solvates,
(2) les sels d'addition d'acide formés avec des acides inorganiques tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphosulfonique, l'acide citrique, l'acide éthanesulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires. Avantageusement, il s'agit de l'acide chlorhydrique ; ou
(3) les sels formés lorsqu'un proton acide présent dans le composé parent soit est remplacé par un ion métallique, par exemple un ion de métal alcalin, un ion de métal alcalino-terreux ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium. Avantageusement, le proton acide est déplacé par un ion Na⁺, notamment en utilisant de l'hydroxyde de sodium. Les sels d'addition d'acide sont formés en particulier avec une fonction amine ou avec une pyridine. Les sels d'addition de base sont formés en particulier avec une fonction acide carboxylique (-COOH), phosphate (-OP(O)(OH)₂) ou encore sulfate (-OSO₃H).

Le terme « stéréoisomères », tel qu'utilisé dans la description de la présente invention, désigne des diastéréoisomères ou des énantiomères. Il s'agit donc d'isomères de configuration. Les stéréoisomères qui ne sont pas des images dans un miroir l'un de l'autre sont ainsi désignés par « diastéréoisomères », et les stéréoisomères qui sont des images l'un de l'autre dans un miroir mais non superposables sont désignés par « énantiomères », encore appelés « isomères optiques ». Un atome de carbone lié à quatre substituants non identiques est appelé un « centre chiral ». Lorsqu'une molécule possède un tel centre chiral, elle est dite chirale et possède deux formes énantiomères. Lorsqu'une molécule possède plusieurs centres chiraux, alors elle possédera plusieurs formes diastéréoisomères et énantiomères. Un mélange équimolaire de deux énantiomères est appelé mélange racémique.

Le terme "prodrogue", tel qu'utilisé dans la description de la présente invention, désigne un composé qui est administré sous une forme inactive (ou moins active) et qui est métabolisé *in vivo,* notamment par action d'enzymes ou de l'acide gastrique, en une forme active (ou plus active). L'utilisation d'une prodrogue permet d'améliorer en particulier les paramètres physico-chimiques d'une molécule tels que la solubilité ainsi que la pharmaco-cinétique (vectorisation, biodisponibilité, etc.), afin de favoriser son assimilation par un organisme après administration.

Selon l'invention, une prodrogue d'une molécule portant un groupement amino (NH₂) pourra résulter notamment de l'acylation ou de la phosphorylation de ce groupement amino. Lorsqu'une molécule porte un groupement hydroxy (OH), la prodrogue pourra résulter en particulier de l'acylation ou de la phosphorylation de ce groupement hydroxy.

L'expression « composés de la présente invention » telle qu'utilisée dans la présente description désigne des composés de formule (Ib) ou (Ic) tels que définis dans les revendications.

### DESCRIPTION DETAILLEE DE L'INVENTION

Les inventeurs ont mis en évidence que les composés de la présente invention perturbent l'assemblage de la tubuline en microtubules. Ces composés possèdent des activités cytotoxiques nanomolaires sur diverses lignées cancéreuses humaines comprenant également des lignées résistantes aux traitements usuels. Ces composés inhibent la polymérisation de la tubuline à des concentrations micromolaires, voire sub-micromolaires, constituant ainsi des composés de choix en thérapeutique.

Du fait même de leur structure chimique, les composés de la présente invention présentent des risques réduits d'être métabolisés à la différence des dérivés de la CA-4 qui sont sujets à (i) des réactions de déméthylation sur le cycle 3,4,5-trimétoxyphényle ou à (ii) l'isomérisation de la double liaison conduisant à une perte notoire d'activité.

### Composés de la présente invention

L'invention a donc pour objet des composés de formule (Ib) telle que définie dans la revendication 1 et qui est liée à la formule (I) suivante : 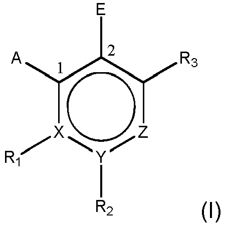 dans laquelle :
- R₂ et R₃ sont différents et un des deux représente un groupement A₁ de formule suivante : 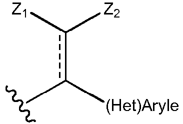 dans lequel :
   - la liaison en pointillé est présente ou absente, de préférence présente ;
   - Z₁ et Z₂ représentent indépendamment l'un de l'autre un atome d'hydrogène, un atome d'halogène ou un groupe méthyle ;
   - (Het)Aryle représente un groupe aryle ou hétéroaryle, ledit hétéroaryle étant choisi parmi les groupes indolyle, benzothiophényle et benzofuranyle, ledit aryle ou hétéroaryle pouvant être substitué par un ou plusieurs substituants choisis parmi un atome d'halogène, un groupe hydroxyle, -OMe, -SMe, - OCX₃ avec X désignant un atome d'halogène, -NH₂, 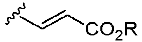 avec R désignant un groupe alkyle en C₁ à C₆, 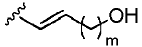 avec m représentant 1 ou 2, 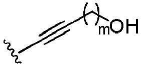 avec m représentant 1 ou 2, et 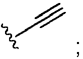
   l'autre représentant :
   - un atome d'hydrogène ;
   - un atome d'halogène ;
   - un groupe hydroxyle ;
   - un groupe cyano ;
   - un groupe -COYR' avec Y désignant O ou N et R' désignant H ou un groupe alkyle en C₁ à C₆, un groupe alcényle en C₂ à C₄, un groupe alcynyle en C₂ à C₄ ;
   - un groupe -SO₂NR'R" avec R', R", désignant chacun, indépendamment l'un de l'autre, H ou un groupe alkyle en C₁ à C₆, un groupe alcényle en C₂ à C₄, un groupe alcynyle en C₂ à C₄ ;
   - un groupe -NHSO₂R' avec R' désignant un groupe alkyle en C₁ à C₆, un groupe alcényle en C₂ à C₄, un groupe alcynyle en C₂ à C₄, un groupe aryle, un groupe hétéroaryle ;
   - un groupe alkyle en C₁ à C₆ ;
   - un groupe alcényle en C₂ à C₄ ;
   - un groupe alcynyle en C₂ à C₄;
   - un groupe alkoxy en C₁ à C₆ ; ou
   - un groupe -NR'R" avec R' et R" représentant, indépendamment l'un de l'autre un hydrogène ou un groupe alkyle ;
- A et E représentent chacun un atome d'hydrogène ou A et E participent à la structure d'un cycle aromatique accolé de formule suivante : 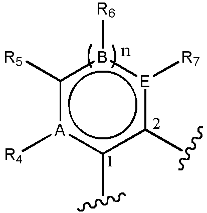 dans laquelle A, B et E représentent, indépendamment l'un de l'autre, un atome de carbone ou d'azote, n représente 0 ou 1 et R₄, R₅, R₆, R₇ sont tels que décrits ci-dessous ;
- X, Y et Z représentent, indépendamment l'un de l'autre, un atome de carbone ou d'azote à la condition que si X et Z représentent un atome d'azote, Y représente un atome de carbone ;
- au moins un de A, B, E, X, Y et Z représente un atome d'azote ;
- R₁, R₄, R₅, R₆, R₇, si présents, représentent, indépendamment l'un de l'autre :
   - un atome d'hydrogène ;
   - un atome d'halogène ;
   - un groupe hydroxyle ;
   - un groupe alkyle en C₁ à C₆ ;
   - un groupe alcényle en C₂ à C₄ ;
   - un groupe alcynyle en C₂ à C₄;
   - un groupe alkoxy en C₁ à C₆ ; ou
   - un groupe -NR'R" avec R' et R" représentant, indépendamment l'un de l'autre un hydrogène ou un groupe alkyle en C₁ à C₆ ;
ainsi que ses sels pharmaceutiquement acceptables, ses stéréoisomères et ses prodrogues.

La formule (Ib) de l'invention est aussi liée à la formule (I') suivante : 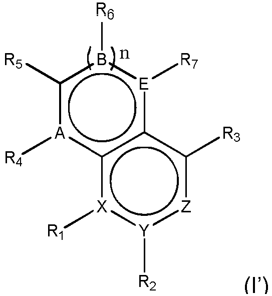 dans laquelle :
- n représente 0 ou 1 ;
- A, B et E représentent, indépendamment l'un de l'autre, un atome de carbone ou d'azote ;
- X, Y et Z représentent, indépendamment l'un de l'autre, un atome de carbone ou d'azote à la condition que si X et Z représentent un atome d'azote, Y représente un atome de carbone ;
- au moins un de A, B, E, X, Y et Z représente un atome d'azote ;
- R₁, R₄, R₅, R₆, R₇, si présents, représentent, indépendamment l'un de l'autre :
   - un atome d'hydrogène ;
   - un atome d'halogène ;
   - un groupe hydroxyle ;
   - un groupe alkyle en C₁ à C₆ ;
   - un groupe alcényle en C₂ à C₄ ;
   - un groupe alcynyle en C₂ à C₄;
   - un groupe alkoxy en C₁ à C₆ ; ou
   - un groupe -NR'R" avec R' et R" représentant, indépendamment l'un de l'autre un hydrogène ou un groupe alkyle en C₁ à C₆ ;
- R₂ et R₃ sont différents et un des deux représente un groupement A₁ de formule suivante : 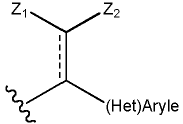 dans lequel :
   - la liaison en pointillé est présente ou absente, de préférence présente ;
   - Z₁ et Z₂ représentent indépendamment l'un de l'autre un atome d'hydrogène, un atome d'halogène ou un groupe méthyle ;
   - (Het)Aryle représente un groupe aryle ou hétéroaryle, ledit hétéroaryle étant choisi parmi les groupes indolyle, benzothiophényle et benzofuranyle, ledit aryle ou hétéroaryle pouvant être substitué par un ou plusieurs substituants choisis parmi un atome d'halogène, un groupe hydroxyle, -OMe, -SMe, - OCX₃ avec X désignant un atome d'halogène, -NH₂, 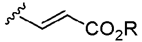 avec R désignant un groupe alkyle en C₁ à C₆, 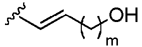 avec m représentant 1 ou 2, 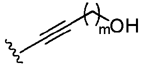 avec m représentant 1 ou 2, et 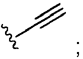
   et l'autre représente :
   - un atome d'hydrogène ;
   - un atome d'halogène ;
   - un groupe hydroxyle ;
   - un groupe cyano ;
   - un groupe -COYR' avec Y désignant O ou N et R' désignant H ou un groupe alkyle en C₁ à C₆, un groupe alcényle en C₂ à C₄, un groupe alcynyle en C₂ à C₄ ;
   - un groupe -SO₂NR'R" avec R', R", désignant chacun, indépendamment l'un de l'autre, H ou un groupe alkyle en C₁ à C₆, un groupe alcényle en C₂ à C₄, un groupe alcynyle en C₂ à C₄ ;
   - un groupe -NHSO₂R' avec R' désignant un groupe alkyle en C₁ à C₆, un groupe alcényle en C₂ à C₄, un groupe alcynyle en C₂ à C_{4,} un groupe aryle, un groupe hétéroaryle ;
   - un groupe alkyle en C₁ à C₆ ;
   - un groupe alcényle en C₂ à C₄ ;
   - un groupe alcynyle en C₂ à C₄;
   - un groupe alkoxy en C₁ à C₆ ;
   - un groupe -NR'R" avec R' et R" représentant, indépendamment l'un de l'autre un hydrogène ou un groupe alkyle en C₁ à C₆ ;
ainsi que ses sels pharmaceutiquement acceptables, ses stéréoisomères et ses prodrogues.

L'expression « R₁, R₄, R₅, R₆ ou R₇, si présents » telle qu'utilisée dans la description de la présente invention signifie que les groupements R₁, R₄, R₅, R₆ ou R₇ seront présents si la valence de l'atome auquel le groupement est, ou serait, lié le permet. L'homme du métier sera aisément déterminé si un tel groupement est présent.

L'invention a pour objet des composés de formule (I') telle que représentée ci-dessus dans laquelle R₃ est un groupement A₁ tel que représenté ci-dessus, soit des composés de formule (Ib) suivante : 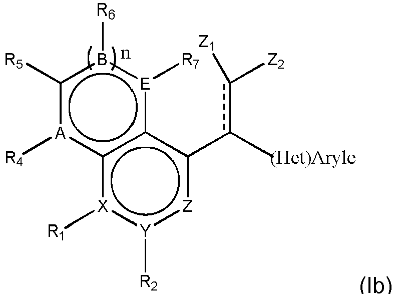 dans laquelle :
- n, A, B, E, X, Y, Z, R₁, R₄, R₅, R₆, R₇, (Het)Aryle, Z₁ et Z₂ sont tels que décrits dans la revendication 1 ; et
- R₂ représente :
   - un atome d'hydrogène,
   - un atome d'halogène,
   - un groupe hydroxyle,
   - un groupe cyano,
   - un groupe -COYR' avec Y désignant O ou N et R' désignant H ou un groupe alkyle en C₁ à C₆, un groupe alcényle en C₂ à C₄, un groupe alcynyle en C₂ à C₄ ;
   - un groupe -SO₂NR'R" avec R', R", désignant chacun, indépendamment l'un de l'autre, H ou un groupe alkyle en C₁ à C₆, un groupe alcényle en C₂ à C₄, un groupe alcynyle en C₂ à C₄ ;
   - un groupe -NHSO₂R' avec R' désignant un groupe alkyle en C₁ à C₆, un groupe alcényle en C₂ à C₄, un groupe alcynyle en C₂ à C_{4,} un groupe aryle, un groupe hétéroaryle;
   - un groupe alkyle en C₁ à C₆,
   - un groupe alcényle en C₂ à C₄,
   - un groupe alcynyle en C₂ à C₄,
   - un groupe alkoxy en C₁ à C₆
   - ou un groupe -NR'R" avec R' et R" représentant, indépendamment l'un de l'autre un hydrogène ou un groupe alkyle.

Dans certains modes de réalisation, l'invention a pour objet des composés de formule (Ib) dans laquelle le groupement (Het)Aryle représente un groupement phényle pouvant être substitué par un ou plusieurs substituants choisis parmi un atome d'halogène, un groupe hydroxyle, -OMe, -SMe, -OCX₃ avec X désignant un atome d'halogène, -NH₂, 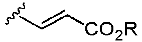 avec R désignant un groupe alkyle en C₁ à C₆, 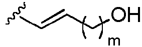 avec m représentant 1 ou 2, 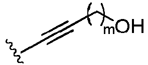 avec m représentant 1 ou 2 et

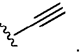

De préférence, le groupement (Het)Aryle représente un groupement phényle substitué présentant la formule suivante : 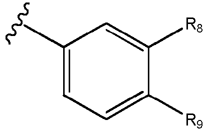 dans laquelle :
- R₈ représente un atome d'hydrogène, un atome d'halogène, un groupe hydroxyle, - OMe, -SMe, -NH₂, 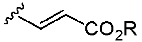 avec R désignant un groupe alkyle en C₁ à C₆, 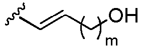 avec m représentant 1 ou 2, 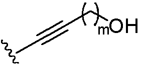 avec m représentant 1 ou 2 ou 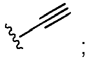 et
- R₉ représente un groupement alkoxy en C₁-C₆, en particulier -OMe, -SMe ou -OCX₃ avec X désignant un atome d'halogène.

De préférence, R₈ représente un atome d'hydrogène, un atome d'halogène, de préférence le fluor ou le chlore, un groupe hydroxyle ou un groupe -NH₂.

De préférence, R₉ représente un groupement alkoxy en C₁-C₆, de préférence en C₁-C₄, de préférence méthoxy, -SMe ou -OCX₃ avec X désignant un atome d'halogène, de préférence le fluor.

Ainsi, dans certains modes de réalisation, l'invention a pour objet des composés de formule (Ic) suivante : 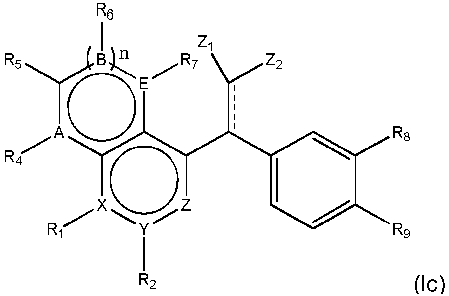 dans laquelle :
- n, A, B, E, X, Y, Z, R₁, R₄, R₅, R₆, R₇ sont tels que définis dans les revendications ;
- R₂ représente :
   - un atome d'hydrogène,
   - un atome d'halogène,
   - un groupe hydroxyle,
   - un groupe cyano,
   - un groupe -COYR' avec Y désignant O ou N et R' désignant H ou un groupe alkyle en C₁ à C₆, un groupe alcényle en C₂ à C₄, un groupe alcynyle en C₂ à C₄ ;
   - un groupe -SO₂NR'R" avec R', R", désignant chacun, indépendamment l'un de l'autre, H ou un groupe alkyle en C₁ à C₆, un groupe alcényle en C₂ à C₄, un groupe alcynyle en C₂ à C₄ ;
   - un groupe -NHSO₂R' avec R' désignant un groupe alkyle en C₁ à C₆, un groupe alcényle en C₂ à C₄, un groupe alcynyle en C₂ à C_{4,} un groupe aryle, un groupe hétéroaryle ;
   - un groupe alkyle en C₁ à C₆,
   - un groupe alcényle en C₂ à C₄,
   - un groupe alcynyle en C₂ à C₄,
   - un groupe alkoxy en C₁ à C₆
   - ou un groupe -NR'R" avec R' et R" représentant, indépendamment l'un de l'autre, un hydrogène ou un groupe alkyle ;
- la liaison en pointillé est absente ou présente, de préférence présente ;
- Z₁ et Z₂ représentent indépendamment l'un de l'autre un atome d'hydrogène, un atome d'halogène ou un groupe méthyle ;
- R₈ représente un atome d'hydrogène, un atome d'halogène, un hydroxyle, -OMe, - SMe, -NH₂, 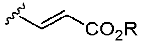 avec R désignant un groupe alkyle en C₁ à C₆, 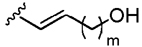 avec m représentant 1 ou 2, 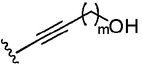 avec m représentant 1 ou 2 ou 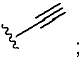 et
- R₉ représente un groupement alkoxy en C₁-C₆, en particulier -OMe, -SMe ou -OCX₃ avec X désignant un atome d'halogène ;
ainsi que ses sels pharmaceutiquement acceptables, ses stéréoisomères et ses prodrogues.

De préférence, dans la formule (Ic) ci-dessus, R₈ représente un atome d'hydrogène, un atome d'halogène, de préférence le fluor ou le chlore, un groupe hydroxyle, un groupe -NH₂, ou 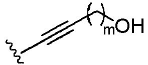 avec m représentant 1 ou 2.

De préférence, dans la formule (Ic) ci-dessus, R₉ représente un groupement alkoxy en C₁-C₆, de préférence en C₁-C₄, de préférence méthoxy, -SMe ou -OCX₃ avec X désignant un atome d'halogène, de préférence le fluor.

De préférence, les composés de la présente invention présentent la formule (Ib) ou (Ic) dans laquelle Z₁ et Z₂ sont identiques et représentent un atome d'hydrogène ou de fluor.

Selon l'invention, la liaison en pointillé est présente.

Dans certains modes de réalisation, les composés de la présente invention présentent la formule (I') représentée ci-dessus et liée à la formule (Ib) de la revendication 1 dans laquelle :
- n, A, B, E X, Y, Z, R₁, R₂, R₄, R₅, R₆, R₇, sont tels que définis dans les revendications ; et
- R₃ représente un groupement A₂ de formule suivante : 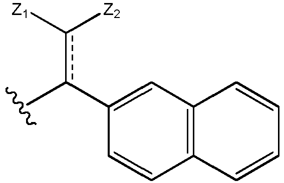 dans lequel :
   - la liaison en pointillé est présente ;
   - Z₁ et Z₂ représentent indépendamment l'un de l'autre un atome d'hydrogène, un atome d'halogène ou un groupe méthyle.

Dans certains modes de réalisation, les composés de la présente invention présentent la formule (I') représentée ci-dessus et liée à la formule (Ib) de la revendication 1 dans laquelle :
- n, A, B, E X, Y, Z, R₁, R₂, R₄, R₅, R₆, R₇, sont tels que définis dans les revendications ; et
- R₃ représente un groupement A₃ de formule suivante : 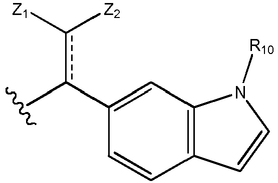 dans lequel :
   - la liaison en pointillé est présente ;
   - Z₁ et Z₂ représentent indépendamment l'un de l'autre un atome d'hydrogène, un atome d'halogène ou un groupe méthyle ;
   - R₁₀ représente un atome d'hydrogène ou un groupe méthyle.

Les composés de la présente invention présentent la formule (Ib) ou (Ic) représentée ci-dessus dans laquelle n est égal à 1, X est un atome d'azote et A, E, Z, Y et B sont un atome de

De préférence, lorsque n est égal à 1, que X est un atome d'azote et que A, E, Z, Y et B sont un atome de carbone, les composés de la présente invention présentent la formule (Ib) ou (Ic) représentée ci-dessus dans laquelle R₄, R₅, R₆ et R₇ représentent un atome d'hydrogène. Dans certains modes de réalisation, les composés de la présente invention présentent la formule (Ib) ou (Ic) dans laquelle n est égal à 1, X est un atome d'azote, A, E, Z, Y et B sont un atome de carbone, R₄, R₅, R₆ et R₇ représentent un atome d'hydrogène et R₂ est tel que défini ci-dessus. De préférence, R₂ est différent d'un atome d'hydrogène.

Dans certains modes de réalisation, les composés de la présente invention présentent la formule (Ib) ou (Ic) représentée ci-dessus dans laquelle :
- n est égal à 1 ;
- A, B, E, Y et Z sont un atome de carbone ;
- X est un atome d'azote.

Dans ces modes de réalisation, de préférence, R₄, R₅, R₆ et R₇ représentent un atome d'hydrogène.

En particulier, les composés de la présente invention peuvent présenter la formule (Ib) ou (Ic) représentée ci-dessus dans laquelle n est égal à 1, A, B, E, Y et Z sont un atome de carbone, X est un atome d'azote et R₂ est tel que défini ci-dessus, de préférence R₂ est différent d'un atome d'hydrogène.

Dans certains modes de réalisation particuliers, les composés de la présente invention présentent la formule suivante: 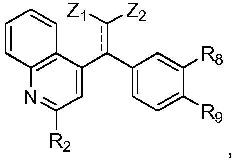 dans lesquelles, de manière appropriée, Z₁, Z₂, R₁, R₂, R₃, R₄, R₅, R₈ et R₉ sont tels que définis ci-dessus et la liaison en pointillé est présente.

En particulier, dans certains modes de réalisation, Z₁ et Z₂ représentent chacun un hydrogène ou chacun un atome de fluor et R₁, R₂, R₃, R₄, R₅, R₈ et R₉ sont tels que définis ci-dessus. Dans d'autres modes de réalisation, R₉ désigne un méthoxy et Z₁, Z₂, R₁, R₂, R₃, R₄, R₅ et R₈ sont tels que définis ci-dessus.

Dans d'autres modes de réalisation, Z₁ et Z₂ représentent chacun un hydrogène ou chacun un atome de fluor, R₉ représente un méthoxy et R₁, R₂, R₃, R₄, R₅ et R₈ sont tels que définis ci-dessus.

En particulier, les composés de l'invention pourront être choisis parmi les composés I-16 et I-19 ci-dessous. Les autres composés ne font pas partie de l'invention :

| | | | |
|---|---|---|---|
| I-1 | 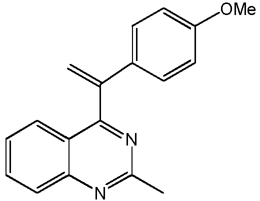 | I-2 | 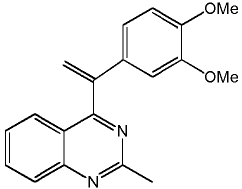 |
| I-3 | 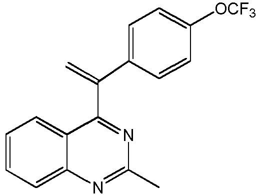 | I-4 | 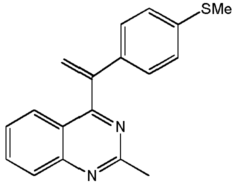 |
| I-5 | 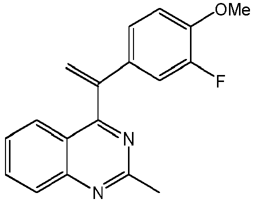 | I-6 | 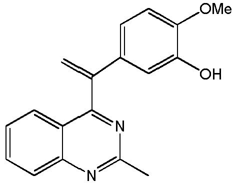 |
| I-7 | 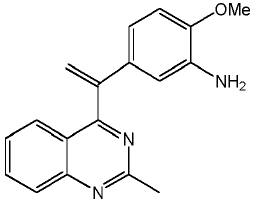 | I-8 | 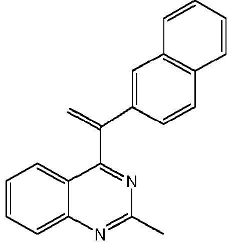 |
| I-9 | 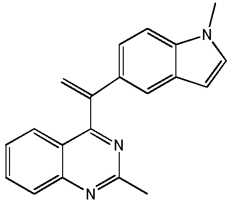 | I-10 | 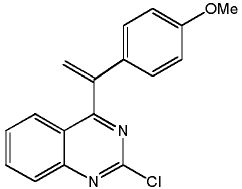 |
| I-11 | 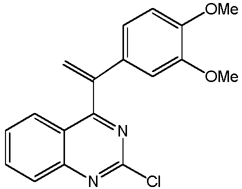 | I-12 | 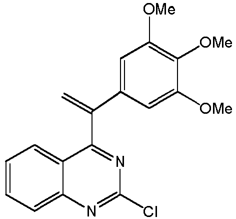 |
| I-13 | 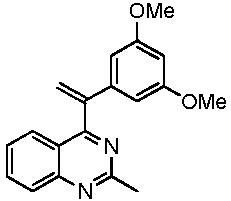 | I-14 | 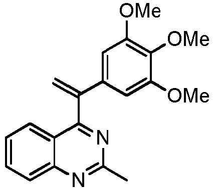 |
| I-15 | 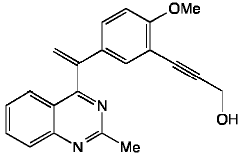 | I-16 | 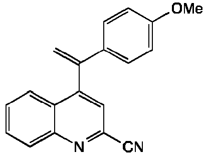 |
| I-17 | 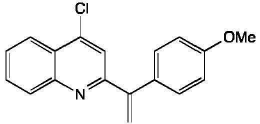 | I-18 | 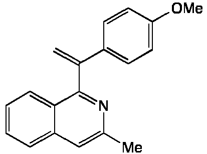 |
| I-19 | 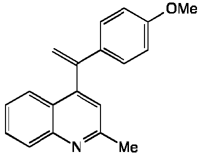 | | |
| II-1 | 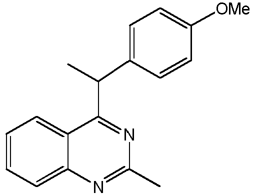 | II-2 | 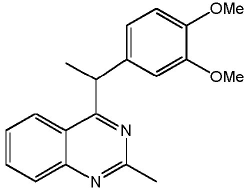 |
| II-3 | 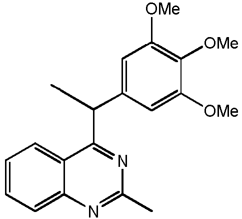 | II-4 | 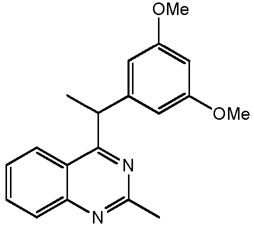 |

L'absence de double liaison éthylénique des composés de formule (I) permet de résoudre le problème d'isomérisation susceptible d'intervenir *in vivo,* entraînant des chutes (ou des absences) d'activité cytotoxique comme c'est par exemple le cas de la CA-4. La réduction de la double liaison située entre les deux noyaux aromatiques conduit à des dérivés moins cytotoxiques que leurs précurseurs.

### Procédé de synthèse des composés de la présente invention

L'invention a également pour objet les procédés de synthèse des composés de la présente invention. Elle est définie par la revendication 14 ci-jointe.

Les procédés de synthèse sont courts, comprenant avantageusement 2 à 3 étapes. Ces procédés sont compatibles avec les exigences industrielles.

Le couplage d'une tosylhydrazone, aisément accessible, avec un dérivé halogéné portant le bicycle azoté conduit aux composés selon l'invention avec d'excellents rendements, avantageusement sans avoir recours à des étapes de protection-déprotection.

Les composés selon l'invention peuvent être préparés selon des procédés connus de l'homme du métier, à partir de produits disponibles dans le commerce ou préparés selon des méthodes connues de l'homme du métier.

En particulier, les composés de formule (I), (I'), (Ib) et (Ic) peuvent être préparés par un procédé comprenant les étapes successives suivantes :
a) réaction d'un composé de formule (II) 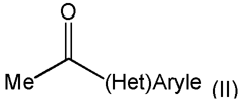 avec la tosylhydrazine pour conduire au composé tosylhydrazone de formule (III) 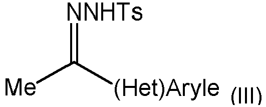
b) couplage du composé de formule (III) avec le composé de formule (IV), (V), (VI) ou (VII) 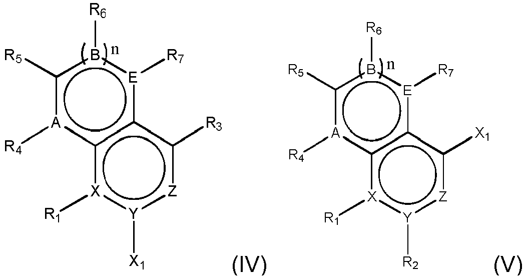 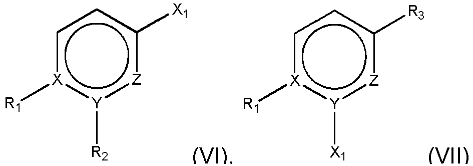
dans laquelle R₁, R₂, R₃, R₄, R₅, R₆, R₇, X, Y, Z, A, B, E et n sont tels que définis précédemment et X₁ représente un atome d'halogène ou trifluorométhanesulfonate ;
b) le cas échéant, réduction du composé (I), dans laquelle la double liaison en pointillée est présente, obtenu suite à l'étape b) pour conduire au composé (I) dans lequel la double liaison en pointillée est absente ;
c) séparation du milieu réactionnel du composé (I) obtenu suite à l'étape b)

L'homme du métier saura protégé de manière appropriée, si nécessaire, les groupements
R₁ à R₇ pour que seul le groupement X₁ réagisse ou adaptée en conséquence la stratégie de synthèse.

Par exemple, lorsque les groupements R₁, R₂, R₃, R₄, R₅, R₆ et/ou R₇ (si présents) représentent un atome d'halogène :
- X₁ est un atome d'iode si les groupements R₁, R₂, R₃, R₄, R₅, R₆ et R₇ (si présents) sont un atome de brome, de chlore ou de fluor ;
- si les groupements R₁, R₂, R₃, R₄, R₅, R₆ et R₇ (si présents) sont un atome de chlore ou de fluor, alors X₁ est un atome de brome.

En particulier, les composés de formule (I'), (Ib) ou (Ic) dans laquelle n est égal à 1, A, B, E et Y sont des atomes de carbone et X et Z sont des atomes d'azote peuvent être préparés par un procédé comprenant les étapes successives suivantes :
a) réaction d'un composé de formule (II) 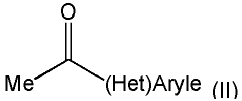 avec la tosylhydrazine pour conduire au composé tosylhydrazone de formule (III) 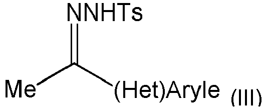
b) couplage du composé de formule (III) avec le composé de formule (IV') ou (V') 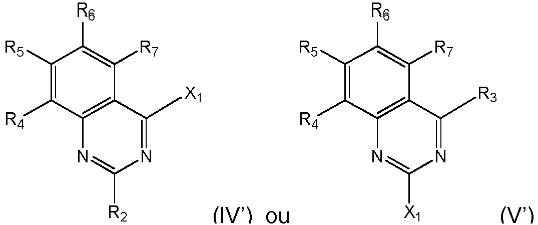 dans laquelle R₂, R₃, R₄, R₅, R₆ et R₇ sont tels que définis précédemment et X₁ représente un atome d'halogène ou trifluorométhanesulfonate ;
c) le cas échéant, réduction du composé (I), dans laquelle la double liaison en pointillée est présente, obtenu suite à l'étape b) pour conduire au composé (I) dans lequel la double liaison en pointillée est absente ;
d) séparation du milieu réactionnel du composé (I) obtenu suite à l'étape b) ou c).

Cette étape c) peut être suivie d'éventuelles étapes supplémentaires classiques de modification des substituants du groupement (Het)Aryle et éventuellement de Z₁/Z₂.

Le composé ainsi obtenu pourra être séparé du milieu réactionnel par des méthodes bien connues de l'homme du métier, comme par exemple par extraction, évaporation du solvant ou encore par précipitation et filtration.

Le composé pourra être par ailleurs purifié si nécessaire par des techniques bien connues de l'homme du métier, comme par recristallisation si le composé est cristallin, par distillation, par chromatographie sur colonne sur gel de silice ou encore par chromatographie liquide haute performance (HPLC).

Lors de l'étape b), le couplage est avantageusement réalisé en présence d'un complexe de palladium, d'un ligand, en quantité catalytique et d'une base. En particulier, le couplage peut être réalisé au moyen d'un complexe de palladium tel que PdCl₂(MeCN)₂], du ligand dppf en quantité catalytique et de la base tBuOLi. Tout particulièrement, le couplage peut être réalisé en présence de 10% molaire de PdCl₂(MeCN)₂, 20% molaire de dppf, 2.2 équivalent molaire de tBuOLi en présence de dioxane, à 90°C, pendant 2 à 4h

Lors de l'étape c), la réduction se fait avantageusement par hydrogénation. L'hydrogénation est avantageusement réalisée sous atmosphère d'hydrogène, notamment en présence de palladium sur charbon (Pd/C) comme catalyseur ou éventuellement de PtO₂. Avantageusement, 5 à 30 mol%, de préférence environ 10 mol% de catalyseur sont utilisés lors de cette réaction. De plus, l'acétate d'éthyle sera avantageusement utilisé comme solvant lors de cette étape.

En particulier, les composés de formule (I), (I'), (Ib) et (Ic) peuvent être préparés par un procédé comprenant les étapes successives suivantes :

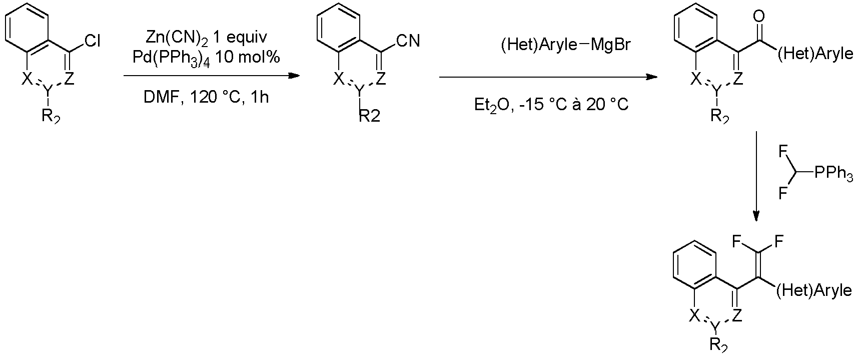

En particulier, certains composés de la présente invention peuvent être préparés selon le schéma suivant :

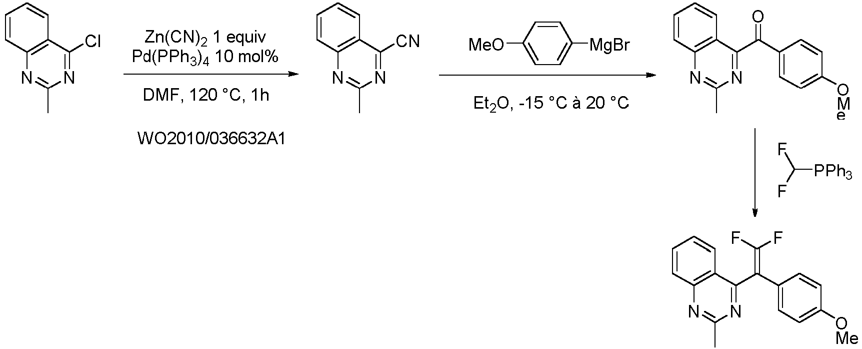

### Utilisations des composés de la présente invention

Dans certains aspects, l''invention porte sur des composés de formule (Ib) ou (Ic), ainsi que leurs sels pharmaceutiquement acceptables, leurs stéréoisomères et leurs prodrogues, pour leur utilisation en tant que médicament.

En particulier, les composés de la présente invention peuvent être utilisés en tant que médicaments inhibiteurs de la polymérisation de la tubuline, avantageusement en tant que médicaments destinés à traiter ou à prévenir les maladies prolifératives, telles que le cancer, le psoriasis ou la fibrose, en particulier le cancer. En particulier, les composés de l'invention, pourront être utiles dans le traitement d'un cancer, tel que ceux susceptibles d'être traités par CA-4 ou par le taxotère.

Les composés de la présente invention peuvent également être utilisés en tant que médicaments anti-vasculaires.

L'invention porte également sur une composition pharmaceutique comprenant au moins un composé selon la présente invention, typiquement en association avec un ou plusieurs excipients pharmaceutiquement acceptables.

Dans certains aspects, l'invention porte également sur une composition pharmaceutique comprenant au moins un composé selon la présente invention en association avec au moins un autre principe actif, notamment un composé anti-cancéreux, cytotoxique ou non, et un ou plusieurs excipients pharmaceutiquement acceptables.

De manière non limitative, les principes actifs pouvant être associés au composé de la présente invention, peuvent être choisis parmi la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifène, l'octréotide, le lanréotide, l'acide (Z)-3-[2,4-diméthyl-5-(2-oxo-1,2-dihydro-indol-3-ylidèneméthyl)-1H-pyrrol-3-yl]-propionique, l'acide 4-((9-chloro-7-(2,6-difluorophényl)-5H-pyrimidol(5,4-d)(2)benzazépin-2-yl)amino)benzoïque, l'acide 5,6-diméthylxanthénone-4-acétique et l'acide 3-(4-(1,2-diphénylbut-1-ényl)phényl)acrylique.

L'invention concerne également une composition pharmaceutique comprenant au moins un composé selon la présente invention en association avec un anticorps. La composition peut comprendre un ou plusieurs excipients pharmaceutiquement acceptables. L'anticorps permet de cibler la tumeur. En particulier, la composition pharmaceutique peut comprendre au moins un composé selon la présente invention en association avec un anticorps monoclonal. L'association du composé de la présente invention avec l'anticorps peut se faire sous forme de conjugués « anticorps-composé de la présente invention ». L'anticorps et le composé de la présente invention sont typiquement liés de manière covalente par l'intermédiaire d'un lien. L'homme du métier saura déterminer la nature du lien adapté à la liaison du composé de la présente invention avec un anticorps. Ainsi, dans un aspect, la présente invention porte sur un conjugué comprenant un composé selon la présente invention lié de manière covalente à un anticorps.

Les composés et compositions selon l'invention peuvent être administrés par voie orale, sublinguale, parentérale, sous-cutanée, intramusculaire, intraveineuse, transdermique, locale ou rectale.

Les composés selon la présente invention peuvent être utilisés à des doses comprises entre 0,01 mg et 1000 mg par jour, donnés en une seule dose une fois par jour ou de préférence administrés en plusieurs doses tout au long de la journée, par exemple deux fois par jour en doses égales. La dose administrée par jour est avantageusement comprise entre 5 mg et 500 mg, encore plus avantageusement entre 10 mg et 200 mg. Il peut être nécessaire d'utiliser des doses sortant de ces gammes ce dont l'homme du métier peut se rendre compte lui-même.

Les composés selon l'invention peuvent être utilisés pour diminuer ou inhiber la polymérisation de la tubuline, notamment *in vitro* et également *in vivo.*

La présente invention porte également sur une composition pharmaceutique comprenant :
(i) au moins un composé de la présente invention,
(ii) au moins un autre principe actif,
en tant que produits de combinaison pour une utilisation simultanée, séparée ou étalée dans le temps.

Le ou les principes actifs peuvent être tels que cités ci-dessus. Notamment, le principe actif peut être utile pour le traitement de maladies prolifératives telles que le cancer, le psiorasis ou la fibrose, avantageusement un agent anti-cancéreux tel qu'un agent anti-vasculaire, cytotoxique ou anti-angiogénique,

Les compositions pharmaceutiques telles que décrites ci-dessus peuvent être utiles pour le traitement des maladies prolifératives, telles que le cancer, le psoriasis ou la fibrose, en particulier le cancer.

La présente invention concerne enfin les composés de l'invention pour leur utilisation dans une méthode de traitement de maladies prolifératives, telles que le cancer, le psoriasis ou la fibrose, et en particulier le cancer, comprenant l'administration, à un patient en ayant besoin d'un composé de la présente invention seul ou en association, avantageusement synergique, avec au moins un autre principe actif tel que défini ci-dessus.

La présente invention concerne également des compositions pharmaceutiques sous forme de formulations nanoparticulaires. Dans de telles modes de réalisation, les compositions pharmaceutiques comprennent au moins un composé de la présente invention couplé de manière covalente à au moins une molécule d'un composé hydrocarboné à structure squalénique. En particulier, les compositions pharmaceutiques comprennent au moins un composé de la présente invention couplé de manière covalente à au moins une molécule d'un composé hydrocarboné à structure squalénique et un solvant polaire ou de l'eau.

Le composé hydrocarboné à structure squalénique et le couplage covalent de ce composé avec les composés de la présente invention peuvent être tel que décrit dans WO 2006/090029 ou FR 2 924 024.

En particulier, les composés hydrocarbonés à structure squalénique désignent des structures hydrocarbonées linéaires formées d'unités isoprènes, plus particulièrement de 6 unités isoprènes. Par exemple, les composés hydrocarbonés à structure squalénique peuvent être l'acide squalénique et ses dérivés, notamment ses dérivés substitués. Les composés hydrocarbonés à structure squalénique peuvent être porteurs d'une fonction susceptible de réagir avec une fonction présente sur les composés de la présente invention de manière à former un lien covalent entre les deux composés, en particulier un lien covalent de type ester, éther, thiéther, disulfure, phosphate ou amide. De manière alternative, le lien covalent entre les deux composés peut être réalisé au moyen d'un bras de liaison. De tels bras de liaison sont bien connus de l'homme du métier.

L'invention va maintenant être illustrée, de manière non limitative, par les exemples I-16 et I-19 qui suivent ; les autres composés ne faisant pas partie de l'invention :

### EXEMPLES

### Procédure générale pour la synthèse des composés I-1 à I-19

A une solution de *N*-tosylhydrazone (1.5 mmol), t-BuOLi (2.2 mmol), PdCl₂(CH₃CN)₂ (0.1 mmol), et du dppf (0.2 mmol) dans le dioxane (1 mL) est ajouté la 4-chloroquinazoline (1 mmol). Le milieu réactionnel est scellé puis chauffé à 90 °C pendant 2 h avant d'être ramené à température ambiante. La suspension obtenue est filtrée sur une colonne de célite (éluant AcOEt) pour séparer les sels inorganiques. Après évaporation des solvants sous vide, le résidu formé est chromatographié sur une colonne de silice.

4-[1-(4-Methoxy-phenyl)-vinyl]-2-methyl-quinazoline **I-1.** Huile jaune, 50%. TLC: *Rf* 0.3 (Cyclohexane/EtOAc, 7/3). IR (neat, cm⁻¹): 1608, 1554, 1511, 1250, 1180. ¹H NMR (300 MHz, CDCl₃): 7.97 (d, 1H, *J* = 8.5 Hz), 7.87 (d, 1H, *J* = 8.5 Hz), 7.81 (td, 1H, *J* = 8.5 Hz, *J* = 0.9 Hz), 7.41 (td, 1H, *J* = 8.3 Hz, *J* = 0.9 Hz), 7.23 (d, 2H, *J* = 8.9 Hz), 6.82 (d, 2H, *J* = 8.9 Hz), 6.04 (s, 1H), 5.46 (s, 1H), 3.78 (s, 3H), 2.93 (s, 3H). ¹³C NMR (75 MHz, CDCl₃): 169.9, 164.1, 159.8, 151.1, 145.0, 133.9, 131.4, 128.1, 128.0 (2), 127.1, 126.7, 121.7, 116.9, 114.1 (2C), 55.4, 26.8. *m*/*z* MS (ESI⁺): 277 (M + H)⁺.

4-[1-(3,4-Dimethoxy-phenyl)-vinyl]-2-methyl-quinazoline **I-2.** Huile jaune, 48%. TLC: Rf 0.1 (Cyclohexane/EtOAc, 7/3). IR (neat, cm⁻¹): 1554, 1514, 1464, 1326, 1221, 1143. ¹H NMR (300 MHz, CDCl₃): 7.96 (d, 1H, *J* = 8.4 Hz), 7.87-7.78 (m, 2H), 7.41 (t, 1H, *J* = 7.6 Hz), 6.97 (d, 1H, *J* = 1.6 Hz), 6.76-6.68 (m, 2H), 6.03 (s, 1H), 5.48 (s, 1H), 3.84 (s, 3H), 3.41 (s, 3H), 2.93 (s, 3H). ¹³C NMR (75 MHz, CDCl₃): 169.7, 164.1, 151.1, 149.5, 149.1, 145.3, 133.9, 131.8, 128.1, 127.0, 126.7, 121.7, 120.1, 117.4, 111.1, 109.5, 56.0 (2C), 26.8. *m*/*z* MS (APCI⁺): 307 (M + H)⁺. 2-Methyl-4-(1-(4-(trifluoromethoxy)phenyl)vinyl)quinazoline **I-3.** Huile jaune, 78%. TLC: Rf 0.3 (Cyclohexane/EtOAc, 7/3). IR (neat, cm⁻¹): 1554, 1509, 1490, 1254, 1207, 1164. ¹H NMR (300 MHz, CDCl₃): 8.00 (d, 1H, *J* = 8.2 Hz), 7.85-7.82 (m, 2H), 7.49-7.43 (td, 1H, *J* = 7.4 Hz, *J* = 1.1 Hz), 7.36 (d, 2H, *J* = 8.9 Hz), 7.15 (d, 2H, *J* = 8.1 Hz), 6.16 (s, 1H), 5.62 (s, 1H), 2.93 (s, 3H). ¹³C NMR (75 MHz, CDCl₃): 169.2, 164.1, 151.1, 149.3, 144.3, 137.4, 134.3, 128.3 (2C), 128.2, 127.1, 126.7, 121.6, 121.1 (2C), 120.4 (q, *J* = 256.5 Hz), 119.9, 26.8. ¹⁹F NMR (188 MHz, CDCl₃): -58.23. *m*/*z* MS (APCI⁺): 331 (M + H)⁺.

2-Methyl-4-(1-(4-(methylthio)phenyl)vinyl)quinazoline **I-4.** Yellow oil, 43%. TLC: *Rf* 0.4 (Cyclohexane/EtOAc, 7/3). IR (neat, cm⁻¹): 1751, 1554, 1492, 1326, 1215. ¹H NMR (300 MHz, CDCl₃): 7.96 (d, 1H, *J* = 8.4 Hz), 7.83-7.76 (m, 2H), 7.42-7.36 (m, 1H), 7.20 (d, 2H, *J* = 8.6 Hz), 7.14 (d, 2H, *J* = 8.7 Hz), 6.09 (s, 1H), 5.52 (s, 1H), 2.91 (s, 3H), 2.42 (s, 3H). ¹³C NMR (75 MHz, CDCl₃): 169.4, 164.1, 151.1, 145.0, 139.2, 135.4, 133.9, 128.1, 127.0 (2C), 126.9, 126.8, 126.3 (2C), 121.6, 118.1, 26.7, 15.5. *m*/*z* MS (APCI⁺): 293 (M + H)⁺.

4-[1-(3-Fluoro-4-methoxy-phenyl)-vinyl]-2-methyl-quinazoline **I-5.** Huile jaune, 46%. TLC: *Rf* 0.2 (Cyclohexane/EtOAc, 7/3). IR (neat, cm⁻¹): 1615, 1554, 1517, 1275, 1134, 1027. ¹H NMR (300 MHz, CDCl₃): 7.99 (d, 1H, *J* = 8.3 Hz), 7.86-7.80 (m, 2H), 7.46-7.41 (td, 1H, *J* = 7.2 *Hz, J =* 1.0 Hz), 7.13 (dd, 1H, *J* = 12.5 Hz, *J* = 2.2 Hz), 6.96-6.92 (m, 1H), 6.85 (t, 1H, *J* = 8.5 Hz), 6.06 (s, 1H), 5.51 (s, 1H), 3.86 (s, 3H), 2.93 (s, 3H). ¹³C NMR (75 MHz, CDCl₃): 169.3, 164.1, 154.1, 150.9 (d, 1C, *J* = 23.7 Hz), 147.9 (d, 1C, *J* = 11.1 Hz), 144.1, 134.1, 131.9 (d, 1C, *J* = 6.3 Hz), 128.2, 126.9, 126.8, 122.9 (d, 1CH, *J* = 3.3 Hz), 121.6, 118.1, 114.3 (d, 1C, *J* = 19.4 Hz), 113.3 (d, 1C, *J* = 1.9 Hz), 56.4, 26.7. ¹⁹F NMR (188 MHz, CDCl₃): -132.7. *m*/*z* MS (APCI⁺): 295 (M + H)⁺.

2-Methoxy-5-(1-(2-methylquinazolin-4-yl)vinyl)phenol **I-6.** A un mélange contenant la *N-*tosylhydrazone silylée (1.5 mmol), t-BuOLi (2.2 mmol), PdCl₂(CH₃CN)₂ (0.1 mmol) et du dppf (0.2 mmol) dans le dioxane (1 mL) est ajouté la 4-chloroquinazoline (1 mmol). Le milieu réactionnel est scellé puis chauffé à 90 °C pendant 2 h avant d'être ramené à température ambiante. La suspension obtenue est filtrée sur une colonne de célite (éluant AcOEt) pour séparer les sels inorganiques. Après évaporation des solvants sous vide, le résidu formé est dissout dans le MeOH (1 mL) puis K₂CO₃ (2 mmol) est ajouté et le milieu réactionnel est agité à température ambiante pendant 6 h. La suspension ainsi formée est filtrée, les solvants organiques sont évaporés et le résidu formé est chromatographié sur une colonne de silice.

Huile jaune, 34%. TLC: *Rf* 0.1 (Cyclohexane/EtOAc, 7/3). IR (neat, cm⁻¹): 1615, 1554, 1512, 1439, 1279, 1135. ¹H NMR (300 MHz, CDCl₃): 7.98 (d, 1H, *J* = 8.4 Hz), 7.87 (d, 1H, *J* = 8.3 Hz), 7.81 (td, 1H, *J* = 7.1 Hz, *J* = 1.2 Hz), 7.42 (t, 1H, *J* = 7.4 Hz), 6.92 (s, 1H), 6.75 (s, 2H), 6.12 (brs, 1H), 6.04 (s, 1H), 5.46 (s, 1H), 3.87 (s, 3H), 2.90 (s, 3H). ¹³C NMR (75 MHz, CDCl₃): 170.1, 164.0, 150.7, 147.1, 145.9, 145.0, 134.1, 132.3, 127.8, 127.1, 126.9, 121.8, 118.9, 117.4, 113.0, 110.8, 56.1, 26.5. *m*/*z* MS (ESI⁺): 293 (M + H)⁺.

2-Methoxy-5-(1-(2-methylquinazolin-4-yl)vinyl)aniline **I-7.** A un mélange contenant la *N-*tosylhydrazone NH-acétyle (1.5 mmol), *t*-BuOLi (2.2 mmol), PdCl₂(CH₃CN)₂ (0.1 mmol) et du dppf (0.2 mmol) dans le dioxane (1 mL) est ajouté la 4-chloroquinazoline (1 mmol). Le milieu réactionnel est scellé puis chauffé à 90 °C pendant 2 h avant d'être ramené à température ambiante. La suspension obtenue est filtrée sur une colonne de célite (éluant AcOEt) pour séparer les sels inorganiques. Après évaporation des solvants sous vide, le résidu formé est dissout dans le MeOH (1 mL) puis KOH (20 mmol) est ajouté et le milieu réactionnel est scellé puis agité à 100 °C pendant 12 h. La suspension ainsi formée est filtrée, les solvants organiques sont évaporés et le résidu formé est chromatographié sur une colonne de silice.

Huile jaune, 27%. TLC: *Rf* 0.1 (Cyclohexane/EtOAc, 7/3). IR (neat, cm⁻¹): 1614, 1567, 1553, 1441, 1330, 1219. ¹H NMR (300 MHz, CD₃COCD₃): 7.94-7.84 (m, 3H), 7.52-7.46 (m, 1H), 6.76 - 6.72 (m, 2H), 6.54 (dd, 1H, *J* = 8.3 Hz, *J* = 2.3 Hz), 5.96 (s, 1H), 5.33 (s, 1H), 4.41 (brs, 2H), 3.81 (s, 3H), 2.79 (s, 3H). ¹³C NMR (75 MHz, CD₃COCD₃): 170.7, 164.6, 152.1, 148.2, 147.1, 138.5, 134.4, 133.1, 128.9, 127.8, 127.3, 122.9, 116.6, 116.1, 113.0, 111.0, 55.8, 26.6. *m*/*z* MS (APCI⁺): 292 (M + H)⁺.

2-Methyl-4-(1-(naphthalen-2-yl)vinyl)quinazoline **I-8.** Solide blanc, 36%. M.p.: 113.6 °C. TLC: Rf 0.2 (Cyclohexane/EtOAc, 7/3). IR (neat, cm⁻¹): 1614, 1568, 1490, 1325, 1168, 906. ¹H NMR (300 MHz, CDCl₃): 8.01 (d, 1H, *J* = 8.4 Hz), 7.86 (d, 1H, *J* = 8.4 Hz), 7.83-7.77 (m, 3H), 7.68-7.65 (m, 1H), 7.60-7.57 (m, 2H), 7.46-7.34 (m, 3H), 6.28 (s, 1H), 5.68 (s, 1H), 2.98 (s, 3H). ¹³C NMR (75 MHz, CDCl₃): 169.6, 164.1, 151.1, 145.6, 136.1, 134.0, 133.3, 133.1, 128.5, 128.4, 128.0, 127.6, 127.0, 126.8, 126.5, 126.4, 126.4, 124.1, 121.7, 119.3, 26.8. *m*/*z* MS (ESI⁺): 297 (M + H)⁺.

2-Methyl-4-(1-(1-methyl-1H-indol-5-yl)vinyl)quinazoline **I-9.** Huile rougeatre, 46%. TLC: *Rf* 0.2 (Cyclohexane/EtOAc, 7/3). IR (neat, cm⁻¹): 1612, 1565, 1490, 1332, 1246. ¹H NMR (300 MHz, CDCl₃): 8.00 (d, 1H, *J* = 8.4 Hz), 7.91 (dd, 1H, *J* = 8.4 Hz, *J* = 0.7 Hz), 7.83-7.77 (ddd, 1H, *J =* 8.4 Hz, *J* = 6.9 Hz, *J* = 1.4 Hz), 7.50-7.49 (m, 1H), 7.40-7.34 (ddd, 1H, *J* = 8.4 Hz, *J* = 6.7 Hz, *J* = 1.3 Hz), 7.30-7.27 (m, 2H), 7.03 (d, 1H, *J* = 3.1 Hz), 6.41 (d, 1H, *J =* 3.1 Hz), 6.12 (d, 1H, *J =* 0.7 Hz), 5.53 (d, 1H, *J* = 0.7 Hz), 3.77 (s, 3H), 2.99 (s, 3H). ¹³C NMR (75 MHz, CDCl₃): 170.6, 164.1, 151.0, 146.8, 136.7, 133.7, 130.7, 129.7, 128.6, 127.9, 127.4, 126.6, 121.9, 120.6, 119.7, 116.7, 109.5, 101.7, 33.0, 26.8. *m*/*z* MS (APCI⁺): 300 (M + H)⁺.

2-Chloro-4-[1-(4-methoxy-phenyl)-vinyl]-quinazoline **I-10.** Huile brune, 46%. TLC: *Rf* 0.4 (Cyclohexane/EtOAc, 7/3). IR (neat, cm⁻¹): 1673, 1597, 1511, 1246, 1175. ¹H NMR (300 MHz, CDCl₃): 8.0 (dd, 1H, *J =* 8.5 Hz, *J* = 1.1 Hz), 7.90-7.85 (m, 2H), 7.49 (td, 1H, *J =* 8.5 Hz, *J =* 1.1 Hz), 7.23 (d, 2H, *J* = 8.8 Hz), 6.84 (d, 2H, *J* = 8.8 Hz), 6.06 (s, 1H), 5.55 (s, 1H), 3.79 (s, 3H). ¹³C NMR (75 MHz, CDCl₃): 173.1, 160.1, 157.3, 152.7, 144.2, 135.1, 131.1, 128.1 (2C), 128.1, 127.9, 127.5, 122.4, 118.6, 114.3 (2C), 55.4. *m*/*z* MS (APCI⁺): 297 (M + H)⁺.

2-Chloro-4-[1-(3,4-dimethoxy-phenyl)-vinyl]-quinazoline **I-11.** Huile jaune, 19%. TLC: Rf 0.3 (Cyclohexane/EtOAc, 7/3). IR (neat, cm⁻¹): 1665, 1595, 1563, 1514, 1465, 1264, 1143. ¹H NMR (300 MHz, CDCl₃): 8.00 (d, 1H, *J* = 8.9 Hz), 7.91-7.85 (m, 2H), 7.49 (td, 1H, *J* = 8.2 Hz, *J =* 1.2 Hz), 6.95 (d, 1H, *J* = 2.0 Hz), 6.77 (d, 1H, *J* = 8.4 Hz), 6.71 (dd, 1H, *J* = 8.3 Hz, *J* = 2.0 Hz), 6.05 (s, 1H), 5.59 (s, 1H), 3.86 (s, 3H), 3.83 (s, 3H). ¹³C NMR (75 MHz, CDCl₃): 172.8, 157.2, 152.7, 149.7, 149.2, 144.5, 135.2, 131.5, 128.0, 128.0, 127.4, 122.4, 120.2, 119.2, 111.2, 109.6, 56.1, 56.0 . *m*/*z* MS (APCI⁺): 327 (M + H)⁺.

2-Chloro-4-[1-(3,4,5-trimethoxy-phenyl)-vinyl]-quinazoline **I-12.** Solide blanc, 34%. M.p.: 135-136 °C. TLC: *Rf* 0.4 (Cyclohexane/EtOAc, 7/3). IR (neat, cm⁻¹): 1580, 1528, 1401, 1316, 1277, 1243, 1121. ¹H NMR (300 MHz, CDCl₃): 8.01 (d, 1H, *J* = 8.9 Hz), 7.92-7.87 (m, 2H), 7.55-7.49 (m, 1H), 6.52 (s, 2H), 6.09 (s, 1H), 5.66 (s, 1H), 3.85 (s, 3H), 3.66 (s, 6H). ¹³C NMR (75 MHz, CDCl₃): 172.4, 157.2, 153.5 (2C), 152.7, 144.9, 138.8, 135.3, 134.3, 128.1 (2C), 127.3, 122.3, 120.8, 104.5 (2C), 61.1, 56.4 (2C). *m*/*z* MS (ESI⁺): 357 (M + H)⁺.

4-[1-(3,5-Dimethoxy-phenyl)-vinyl]-2-methyl-quinazoline **I-13.** Solide blanc, 36%. F. 89-91 °C. *Rf* 0.3 (Cyclohexane/EtOAc, 7/3). IR (neat, cm⁻¹): 1592, 1460, 1424, 1294, 1206, 1161. ¹H NMR (300 MHz, CDCl₃): 7.96 (d, 1H, *J* = 8.4 Hz), 7.87-7.78 (m, 2H), 7.42 (td, 1H, *J* = 8.1 Hz, *J =* 1.0 Hz), 6.45 (d, 2H, *J* =2.1 Hz), 6.41 (d, 1H, *J* = 2.0 Hz), 6.12 (s, 1H), 5.58 (s, 1H), 3.72 (s, 6H), 2.93 (s, 3H). ¹³C NMR (75 MHz, CDCl₃): 169.3, 164.1, 161.0 (2C), 151.1, 145.7, 141.0, 134.0, 128.1, 127.0, 126.8, 121.7, 119.5, 105.4 (2C), 100.2, 55.5 (2C), 26.8. *m*/*z* MS (APCI⁺): 307 (M + H)⁺.

2-Methyl-4-[1-(3,4,5-trimethoxy-phenyl)-vinyl]-quinazoline **I-14.** Solide jaune, 40%. F. 127-128 °C. *Rf* 0.2 (Cyclohexane/EtOAc, 7/3). IR (neat, cm⁻¹): 1553, 1506, 1464, 1410, 1331, 1242, 1128. ¹H NMR (300 MHz, CDCl₃): 7.97 (d, 1H, *J* = 8.5 Hz), 7.90-7.80 (m, 2H), 7.45 (td, 1H, *J =* 8.2 Hz, *J* = 1.1 Hz), 6.54 (s, 2H), 6.07 (s, 1H), 5.56 (s, 1H), 3.84 (s, 3H), 3.72 (s, 6H), 2.94 (s, 3H). ¹³C NMR (75 MHz, CDCl₃): 169.3, 164.1, 153.4 (2C), 151.2, 145.7, 138.6, 134.7, 134.0, 128.1, 127.0, 126.9, 121.7, 119.0, 104.5 (2C), 61.0, 56.3 (2C), 26.8. *m*/*z* MS (ESI⁺): 359 (M + Na)⁺.

Le composé 3-(2-Méthoxy-5-(1-(2-méthylquinazolin-4-yl)vinyl)phényl)prop-2-yn-1-ol ***I-15*** a été préparé en 3 étapes à partir de la 3-iodo-4-méthoxyacétophénone commerciale (Schéma 1). La première étape est une réaction de Sonogashira permettant d'installer la fonction acétylénique. La cétone ainsi formée est transformée en dérivé N-tosylhydrazone puis couplage de ce dernier dans des conditions pallado-catalysées avec la 4-chloroquinazoline pour fournir le compose **I-15.**

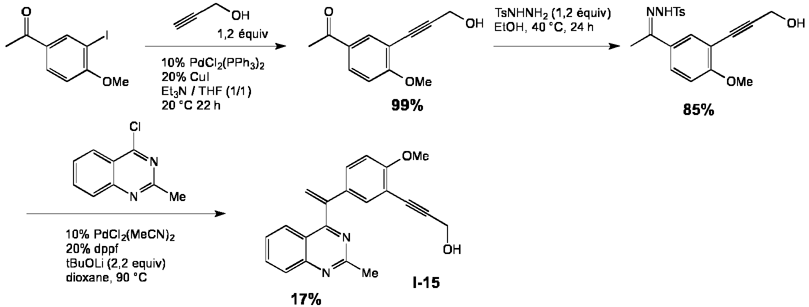

Huile jaune, 17%, TLC : Rf 0.1 (Cyclohexane/EtOAc, 1/1). IR (neat, cm⁻¹): 3272, 2927, 2840, 2247, 1777, 1599, 1361, 873. ¹H NMR (300 MHz, CDCl₃): 8.01 (d, 1H, *J* = 8.3 Hz), 7.82 (m, 2H), 7.46, (t, 1H, *J* = 7.5 Hz), 7.41 (s, 1H), 7.22 (d, 1H, *J* = 7.5 Hz), 6.77 (d, 1H, *J* = 8.5 Hz), 6.02 (s, 1H), 5.50 (s, 1H), 4.50 (s, 2H), 3.78 (s, 3H), 2.90 (s, 3H), OH not seen). ¹³C NMR (300 MHz, CDCl₃): 169.5, 163.9, 160.0, 150.7, 144.1, 134.1, 131.9, 131.2, 128.4, 127.8, 126.9, 126.8, 121.5, 117.9, 112.1, 110.7, 92.0, 81.3, 55.9, 51.6, 26.5. HRMS (ESI⁺): *m*/*z* calculé pour for C₂₁H₁₉N₂O₂ [M+H]⁺ 331.1447; trouvé 331.1441.

Le composé 4-(1-(4-Méthoxyphenyl)vinyl)quinoline-2-carbonitrile **I-16** est préparé à partir de la 2,4-dichloroquinoléine en réalisant d'abord une réaction de cyanation sélective en position C2 (schéma 2). La 2-cyano-4-chloroquinoléine ainsi formé avec un rendement de 83% est ensuite couplé avec la N-tosylhydrazone du 4-méthoxyacétophénone.

Huile jaune, 62%, TLC : Rf 0.4 (Cyclohexane/EtOAc, 9/1). R (neat, cm⁻¹): 3062, 3004, 2958, 2934, 2838, 2236, 1675, 1605, 1576, 1545, 1511, 1459, 1218, 1153, 1098, 909. ¹H NMR (300 MHz, CDCl₃): 8.20 (d, 1H, *J =* 9.1 Hz), 7.91-7.78 (m, 2H), 7.65 (s, 1H), 7.61-7.50 (m, 1H), 7.18 (d, 2H, *J* = 9.0 Hz), 682 (d, 2H, *J* = 9.0 Hz), 6.01 (s, 1H), 5.36 (s, 1H), 3.79 (s, 3H). ¹³C NMR (300 MHz, CDCl₃): 160.1, 150.8, 148.7, 144.3, 133.7, 131.7, 131.1, 130.4, 129.4, 127.9 (2C), 126.4, 123.8, 117.7, 116.6, 114.3 (2C), 55.4. HRMS (ESI⁺): *m*/*z* calculé pour for C₁₉H₁₄N₂O [M+H]⁺ 287.1184; trouvé 287.1180.

### Le composé 4-Chloro-2-(1-(4-méthoxyphenyl)vinyl)quinoléine I-17 est préparé à partir de la 2,4-dichloroquinoléine)

Huile brune, 47%, TLC : Rf 0.5 (Cyclohexane/EtOAc, 9/1). IR (neat, cm⁻¹): 3400, 3063, 3001, 2956, 2933, 2907, 2836, 1939, 1652, 1607, 1440, 1245, 907, 846. ¹H NMR (300 MHz, CDCl₃): 8.25-8.17 (m, 2H), 7.81-7.75 (td, 1H, *J* = 6.9 Hz, *J* = 1.5 Hz), 7.69-7.61 (td, 1H, *J =* 6.9 Hz, *J =* 1.5 Hz), 7.50 (s, 1H), 7.30 (d, 2H, *J* = 9.0 Hz), 6.89 (d, 2H, *J* = 9.0 Hz), 6.04 (d, 1H, *J* = 1.3 Hz), 5.75 (d, 1H, *J =* 1.3 Hz), 3.79 (s, 3H). ¹³C NMR (300 MHz, CDCl₃): 159.8, 159.1, 148.5, 147.9, 143.0, 132.0, 130.8, 130.0, 129.7 (2C), 127.6, 125.7, 124.0, 121.4, 118.7, 114.0 (2C), 55.5. HRMS (ESI⁺): *m*/*z* calculé for C₁₈H₁₅ClNO [M+H]⁺ 296.0842; trouvé 296.0842.

Le composé 1-(1-(4-Méthoxyphényl)vinyl)-3-méthylisoquinoléine **I-18** est préparé à partir de la 1-chloro-3-méthylisoquinoléine) Solide blanc, 40 %, F = 66°C, TLC : Rf 0.42 (Cyclohexane/EtOAc, 3/7). IR (neat, cm⁻¹): 2955, 2835, 1621, 1606, 1589, 1464, 1298, 1285, 1098, 879. ¹H NMR (300 MHz, CDCl₃): 7.82 (d, 1H, *J* = 9.0 Hz), 7.66 (d, 1H, *J* = 9.0 Hz), 7.51 (t, 1H, *J =* 9.0 Hz), 7.40 (s, 1H), 7.27 (t, 1H, *J* = 9.0 Hz), 7.16 (d, 2H, *J* = 9.1 Hz), 6.69 (d, 2H, *J* = 9.1 Hz), 5.94 (s, 1H), 5.31 (s, 1H), 3.69 (s, 3H), 2.65 (s, 3H). ¹³C NMR (300 MHz, CDCl₃): 160.9, 159.4, 150.9, 146.6, 137.2, 132.5, 130.0, 127.8 (2C), 127.4, 126.2, 126.1, 125.4, 118.2, 115.4, 113.7 (2C), 55.2, 24.5. HRMS (ESI⁺): *m*/*z* calculé pour C₁₉H₁₈NO [M+H]⁺ 276.1388; trouvé 276.1385.

### Le composé 4-(1-(4-Méthoxyphényl)vinyl)-2-méthylquinoléine I-19 est préparé à partir de la 4-chloro-2-méthylquinoléine)

Solide blanc, 65 %, F = 94°C, TLC : Rf 0.24 (Cyclohexane/EtOAc, 8/2). IR (neat, cm⁻¹): 2961, 2929, 1734, 1651, 1607, 1560, 1440, 1409, 1378, 1097, 902, 836. ¹H NMR (300 MHz, CDCl₃): 8.04 (d, 1H, *J* = 8.2 Hz), 7.72 (d, 1H, *J* = 8.3 Hz), 7.62 (dd, 1H, *J* = 8.35, *J* = 6.92), 7.22 (t, 1H, *J* = 8.3 Hz), 7.18 (m, 3H), 6.80 (d, 2H, *J* = 8.0 Hz), 5.80 (s, 1H), 5.31 (s, 1H), 3.77 (s, 3H), 2.76 (s, 3H). ¹³C NMR (300 MHz, CDCl₃): 158.5, 157.7, 147.7, 147.1, 144.5, 131.3, 128.2, 127.8, 126.7 (2C), 125.0, 124.5, 124.4, 121.3, 113.9, 112.8 (2C), 54.2, 24.3. HRMS (ESI⁺): *m*/*z* calculé pour C₁₉H₁₈NO [M+H]⁺ 276.1388; trouvé 276.1388.

### Procédure générale pour la synthèse des composés II-1 à II-4

Une solution de 1-quinazoline-1-arylethylènes (**I**) (1 mmol) dans EtOAc (1 mL) est placée sous pression atmospherique d'hydrogène en presence de Pd/C (20% en masse) puis agitée pendant 2 à 5 h. Après filtration sur célite, les solvants sont évaporés sous vide, et le résidu formé est chromatographié sur une colonne de silice.

4-[1-(4-Methoxy-phenyl)-ethyl]-2-methyl-quinazoline **II-1.** Huile brune, 99%. TLC: *Rf* 0.4 (Cyclohexane/EtOAc, 7/3). IR (neat, cm⁻¹): 2925, 1612, 1564, 1511, 1463, 1247, 1178. ¹H NMR (300 MHz, CDCl₃): 8.07 (d, 1H, *J* = 8.4 Hz), 7.91 (d, 1H, *J* = 8.4 Hz), 7.75-7.69 (m, 1H), 7.45-7.39 (m, 1H),7.24 (d, 2H, *J* = 8.7 Hz), 6.79 (d, 2H, *J* = 8.7 Hz), 4.95 (q, 1H, *J* = 6.9 Hz), 3.71 (s, 3H), 2.92 (s, 3H), 1.78 (d, 3H, *J* =6.9 Hz). ¹³C NMR (75 MHz, CDCl₃): 172.4, 163.8, 158.2, 150.7, 136.5, 133.0, 128.6 (2C), 128.5, 126.4, 124.6, 121.4, 114.1 (2C), 55.2, 42.2, 26.8, 21.3. *m*/*z* MS (APCI⁺): 279 (M + H)⁺.

4-[1-(3,4-Dimethoxy-phenyl)-ethyl]-2-methyl-quinazoline **II-2.** Huile jaune, 97%. TLC: *Rf* 0.2 (Cyclohexane/EtOAc, 7/3). IR (neat, cm⁻¹): 1563, 1514, 1491, 1418, 1328, 1262, 1140. ¹H NMR (300 MHz, CDCl₃): 8.10 (d, 1H, *J =* 8.2 Hz), 7.90 (d, 1H, *J =* 8.3 Hz), 7.75 (ddd, 1H, *J* =8.2 Hz, *J* = 6.9 Hz, *J =* 1.2 Hz), 7.45 (ddd, 1H, *J* = 8.2 Hz, *J* = 6.8 Hz, *J* = 1.1 Hz), 6.92 (d, 1H, *J* = 2.0 Hz), 6.86 (dd, 1H, *J* = 8.2 Hz, *J* = 2.0 Hz), 6.75 (d, 1H, *J* = 8.2 Hz), 4.94 (q, 1H, *J* = 6.9 Hz), 3.82 (s, 3H), 3.81 (s, 3H), 2.91 (s, 3H), 1.78 (d, 3H, *J* = 6.9 Hz). ¹³C NMR (75 MHz, CDCl₃): 172.3, 163.8, 150.8, 149.1, 147.8, 137.0, 133.2, 128.6, 126.5, 124.7, 121.4, 119.8, 111.3, 111.0, 56.0, 55.9, 42.6, 26.9, 21.4. *m*/*z* MS (APCI⁺): 309 (M + H)⁺.

2-Methyl-4-[1-(3,4,5-trimethoxy-phenyl)-ethyl]-quinazoline **II-3.** Huile jaune, 97%. *Rf* 0.2 (Cyclohexane/EtOAc, 7/3). IR (neat, cm⁻¹): 1587, 1564, 1492, 1461, 1420, 1328, 1238, 1122. ¹H NMR (300 MHz, CDCl₃): 8.1 (d, 1H, *J* = 8.2 Hz), 7.91 (d, 1H, *J* = 8.3 Hz), 7.76 (ddd, 1H, *J =* 8.2 Hz, *J =* 7.1 Hz, *J* = 1.3 Hz), 7.47 (ddd, 1H, *J* = 8.2 Hz, *J* = 7.0 Hz, *J =* 1.2 Hz), 6.58 (s, 2H), 4.91 (q, 1H, *J* = 6.9 Hz), 3.79 (s, 6H), 3.77 (s, 3H), 2.91 (s, 3H), 1.78 (d, 3H, *J* = 6.9 Hz). ¹³C NMR (75 MHz, CDCl₃): 172.0, 163.8, 153.3 (2C), 150.8, 140.0, 136.8, 133.2, 128.6, 126.6, 124.6, 121.5, 105.0 (2C), 60.9, 56.2 (2C), 43.2, 26.8, 21.5. *m*/*z* MS (APCI⁺): 339 (M + H)⁺.

4-[1-(3,5-Dimethoxy-phenyl)-ethyl]-2-methyl-quinazoline **II-4.** Huile brune, 89%.Rf 0.3 (Cyclohexane/EtOAc, 7/3). IR (neat, cm⁻¹): 1609, 1593, 1564, 1428, 1205, 1157. ¹H NMR (300 MHz, CDCl₃): 8.08 (d, 1H, *J* = 8.2 Hz), 7.90 (d, 1H, *J* = 8.4 Hz), 7.77-7.72 (ddd, 1H, *J* = 8.2 Hz, *J* = 6.9 Hz, *J* = 1.3 Hz), 7.44 (ddd, 1H, *J* = 8.3 Hz, *J* = 7.0 Hz, *J* = 1.0 Hz), 6.49 (d, 2H, *J* = 2.2 Hz), 6.27 (t, 1H, *J =* 2.2 Hz), 4.90 (q, 1H, *J* = 6.9 Hz), 3.73 (s, 6H), 2.91 (s, 3H), 1.78 (d, 3H, *J =* 6.9 Hz). ¹³C NMR (75 MHz, CDCl₃): 171.8, 163.8, 161.0 (2C), 150.7, 146.9, 133.2, 128.5, 126.6, 124.7, 121.6, 106.2 (2C), 98.0, 55.4 (2C), 43.4, 26.9, 21.1. *m*/*z* MS (APCI⁺): 309 (M + H)⁺.

### Protocole pour la cytotoxicité.

Les cellules U87, H1299, MDA-MB231 sont cultivées dans du milieu de culture « Dulbecco minimal essential medium » (DMEM) contenant 4,5 g/L de glucose et supplémenté avec 10 % de sérum de veau fœtal et 1 % de glutamine. Les cellules K562 et HCT116 sont cultivées dans du milieu RPMI 1640 contenant 10 % de sérum de veau fœtal et 1 % de glutamine. Toutes les lignées cellulaires sont maintenues en culture à 37 °C dans une atmosphère humide contenant 5% de CO₂. Les cellules sont ensemencées dans des plaques de culture de 96 puits à raison de 5000 cellules par puits dans 50 µL de milieu de culture. Après 24 h de culture, la molécule à tester, dissoute dans le DMSO et diluée à 1/1000 dans le milieu de culture qui convient, est ajoutée dans chacun des puits à raison de 50 µL par puits. Après 72 h d'incubation, 20 µL de résazurine sont ajoutés dans chaque puits. Après 1 heure d'incubation, la fluorescence émise est mesurée à 590 nm après excitation à 560 nm à l'aide du lecteur de fluorescence Victor (Perkin-Elmer, USA). Chaque concentration est testée trois fois et chaque expérience est renouvelée trois fois.

### Protocole pour l'inhibition de la polymérisation de la tubuline.

Lorsque la tubuline est placée à une température de 0 °C, elle se trouve sous forme de dimères libres. En revanche, lorsque la température augmente brutalement à 37 °C, les dimères s'assemblent en microtubules. La détermination de l'IPT des différents analogues synthétisés a été effectuée sur une tubuline soluble selon la méthode de Shelanski⁷ à partir de cerveaux de mouton où elle constitue 20 à 25% des protéines solubles. L'activité « antitubuline » est évaluée selon la méthode de Gaskin⁸ par mesure de la turbidité qui est proportionnelle à la concentration de microtubules dans la suspension. La turbidité est mesurée par absorption de la suspension à 350 nm à l'aide d'un spectrophotomètre équipé d'une cellule thermostatée à 37 °C. Les différents échantillons ont été dissous dans le DMSO et incubés 10 minutes à 37 °C puis 5 minutes à 0 °C.

Les courbes d'assemblage et de désassemblage de la tubuline sont obtenues par cinétique de l'absorption à 350 nM en fonction du temps. La Cl₅₀ déterminée graphiquement, est définie comme la concentration d'inhibiteur nécessaire pour diminuer de 50% le taux d'assemblage maximal de la tubuline témoin.

### Étude biologique in vitro des composés I-16 et I-19 de la présente invention

Leurs effets sur la prolifération de différentes lignées cellulaires humaines cancéreuses et leur capacité à inhiber la polymérisation de la tubuline a été testés.

### A. Activité cytotoxique

L'activité cytotoxique des composés a été étudiée *in vitro* principalement sur la lignée cellulaire cancéreuse humaine HCT116 *(carcinome colorectal).* La lignée sélectionnée pour cette étude a été incubée à 37 °C en présence de l'un des composés ajouté dans le milieu de culture à différentes concentrations. La concentration inhibitrice induisant la mort de 50% des cellules (Cl₅₀) a été déterminée après 72 heures d'incubation (Tableau 1) pour chaque composé. L'ensemble des expériences réalisées a permis de déterminer le degré de toxicité des composés testés en prenant l'isoCA-4 comme référence. Au vu des résultats, il apparaît que plusieurs composés possèdent des Cl₅₀ à un niveau nanomolaire et que les composés **I-5, I-6, I-7, 1-15, I-16, 1-19** sont les plus cytotoxiques avec des valeurs de Cl₅₀ inférieures à 20 nM. Il est à noter que les premières relations structure-activité sont en parfait accord avec les résultats précédents observés en série isoCA-4 et isoérianine, à savoir que les cycles B les plus efficaces sont substitués en C-4 par un groupement OMe et en C-3 par un atome de fluor (**I-5**), un groupement OH (**I-6**) ou NH₂ (**I-7**). De plus, en comparant les résultats obtenus avec les composés cytotoxiques **I-1** et **I-10**, il apparait que le noyau quinazoline peut être substitué en C-2 par un groupement méthyle ou par un atome de chlore sans perte d'efficacité (**I-1** et **I-10**: Cl₅₀ = 40 nM). Enfin, lorsque l'on examine ces premiers résultats, il apparait que la réduction de la double liaison située entre les deux noyaux aromatiques conduit à des dérivés « réduits » de type **II** environ dix fois moins cytotoxiques que leurs précurseurs I comme nous l'avions déjà constaté lors de la réduction des dérivés *iso*CA-4 en dérivés de type isoérianine. La molécule **I-15** montre que l'introduction d'un substituant carboné acétylénique en C3' améliore considérablement l'activité cytotoxique par rapport à **I-1.** Par ailleurs, le remplacement du noyau quinazoline par d'autres hétérocycles a permis d'identifier des molécules montrant de meilleures activités cytotoxiques à des concentrations sub-nanomolaires.

**Tableau 1 : Résultats de cytotoxicité des composés sur la lignée cellulaire cancéreuse humaine HCT116 (carcinome colorectal). L'activité cytotoxique (Cl₅₀) est exprimée en nMol/L et correspond à la concentration des composés qui induit 50% de mortalité cellulaire après 72 h d'incubation.**

| **Composés** | | **HCT116 Cl₅₀ (nM)** | **Composés** | | **HCT116 Cl₅₀ (nM)** |
|---|---|---|---|---|---|
| 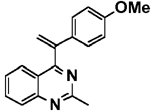 | **I-1** | **38** | 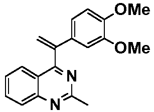 | **I-2** | **460** |
| 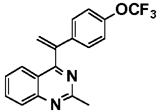 | **I-3** | **2300** | 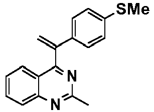 | **I-4** | **52** |
| 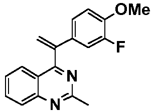 | **I-5** | **18** | 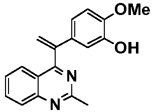 | **I-6** | **10** |
| 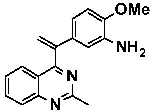 | **I-7** | **10** | 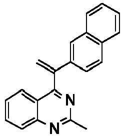 | **I-8** | **130** |
| 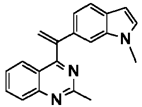 | **I-9** | **27** | 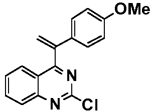 | **I-10** | **34** |
| 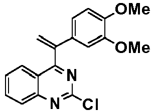 | **I-11** | **700** | 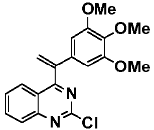 | **I-12** | **900** |
| 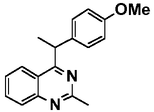 | **II.1** | **250** | 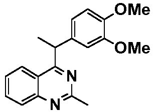 | **II-2** | **950** |
| 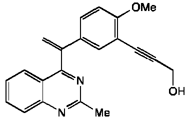 | **I-15** | **1,5** | 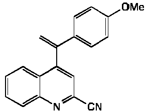 | **I-16** | 1,6 |
| 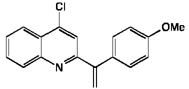 | **I-17** | 95 | 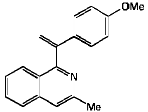 | **I-18** | 87 |
| 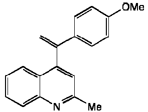 | **I-19** | ≤1 | | | |
| ***iso*CA-4** | | **2** | | | |

Les composés **I-1** et **I-10** porteurs d'un substituant méthyle et d'un atome de chlore en C-2 et qui présentaient des niveaux de cytotoxicité équivalentes (Cl₅₀ = 40 nM) vis-à-vis de la lignée cellulaire HCT116 ont été également évalués sur une lignée de leucémie myéloïde chronique K562 et sur une lignée de cancer du poumon non à petites cellules H1299. Les composés **I-15, I-16 et I-19** ont également été évalués sur une lignée de leucémie myéloïde chronique K562 et sur une lignée de leucémie myéloïde chronique K562R résistante à l'imatinib. Les premiers résultats de cytotoxicité sont rapportés dans le tableau 2 ci-après.

**Tableau 2 : Résultats de cytotoxicité des composés I-1 et I-10 sur 3 lignées cellulaires cancéreuses humaines HCT116 (carcinome colorectal), K562 (leucémie myéloïde chronique) et H1299 (cancer du poumon non à petites cellules). L'activité cytotoxique (Cl₅₀) est exprimée en nMol/L et correspond à la concentration des composés qui induit 50% de mortalité cellulaire après 72 h d'incubation.**

| **Composés** | **HCT116 Cl₅₀ (nM)** | **K562 Cl₅₀ (nM)** | **K562R Cl₅₀ (nM)** | **H1299 Cl₅₀ (nM)** |
|---|---|---|---|---|
| **I-1** | **38** | **34** | **n/a** | **22** |
| **I-10** | **34** | **38** | **n/a** | **25** |
| **I-15** | **1,5** | **8** | **≤ 1** | **n/a** |
| **I-16** | **1,6** | **9** | **≤ 1** | **n/a** |
| **I-19** | **≤ 1** | **17** | **0,7** | **n/a** |
| ***iso*CA-4** | **2** | **4** | | **5** |

Les résultats indiquent que la cytotoxicité de **I-1** et **I-10** vis-à-vis des lignées K562 et H1299 est similaire à celle observée avec la lignée HCT116 (Cl₅₀ comprises entre 22 et 38 nM).

Le remplacement du noyau quinazoline (I-1) par un noyau quinoléine a fourni le composé **1-19** possédant des activités cytotoxiques sub-nanomolaires. Il est à noter que le remplacement du groupement méthyle en C2 du composé **1-19** par un groupement cyano **(1-16)** n'a pas d'influence sur l'activité cytotoxique.

### B Inhibition de la polymérisation de la tubuline

Les composés le plus cytotoxiques ont été sélectionnés pour des tests d'inhibition de la polymérisation de la tubuline. Ces tests ont été effectués sous la direction du Dr J. Dubois à Gif sur Yvette. La tubuline est purifiée à partir de cervelles de brebis selon la méthode de Shelanski⁹ par 2 cycles d'assemblage-désassemblage. La solution mère (15-20 mg/mL), stockée à -196 °C, est décongelée et diluée dans le tampon d'assemblage (0.1 M MES, 0.5 mM MgCl₂, 1 mM EGTA, and 1 mM GTP, pH 6.6) pour avoir une concentration finale de 10 µM. L'assemblage de la tubuline est suivi par fluorescence sur plaques 96-puits selon la méthode de Barron *et* al.¹⁰ A la solution de tubuline (10 µM, 100 µL par puits) est ajouté l'inhibiteur (DMSO, 1 µL) et la solution est incubée 45 min à température ambiante. Le GTP (1 mM final) est ensuite ajouté, la solution est mélangée rapidement et la fluorescence (λₑₓ = 350 nm, λₑₘ = 440 nm) est mesurée sur un fluorimètre Wallac Victor (Perkin Elmer). La détermination de l'inhibition de 50% du taux d'assemblage maximum (Cl₅₀) est réalisée en duplicate ou triplicate sur 10 concentrations encadrant la Cl₅₀. Les résultats sont consignés dans le tableau 3.

**Tableau 3 : Résultats d'inhibition de la polymérisation de la tubuline (IPT) par les composés I sélectionnés**

| **Composés** | | **IPT (Cl₅₀) µM** | **Composés** | | **IPT (Cl₅₀) µM** |
|---|---|---|---|---|---|
| 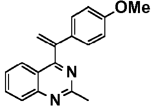 | **I-1** | **1,8** | 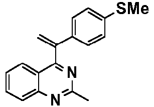 | **I-4** | **2,9** |
| 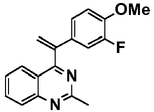 | **I-5** | **1,0** | 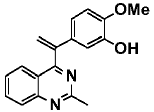 | **I-6** | **0,6** |
| 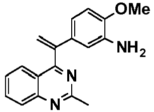 | **I-7** | **2,3** | 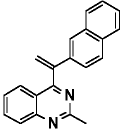 | **I-8** | **2,2** |
| 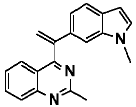 | **I-9** | **1,6** | 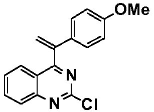 | **I-10** | **1,9** |
| 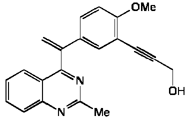 | **I-15** | **1,2** | 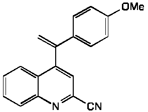 | **I-16** | **0,96** |
| 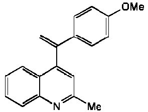 | **I-19** | **1,09** | | | |
| ***iso*CA-4** | | **1,0** | **CA-4** | | **1,0** |

On peut noter que l'ensemble des composés sélectionnés inhibe la polymérisation de la tubuline à des concentrations micromolaires (0,6 µM<CI₅₀<2.9 µM). En particulier, le composé **I-6** qui s'est révélé être très cytotoxique vis-à-vis de la lignée HCT116, inhibe l'assemblage de la tubuline avec une CI₅₀ sub-micromolaire (CI₅₀ = 0.6 µM).

La molécule **I-15** montre que l'introduction d'un substituant carboné acétylénique en C3' améliore considérablement l'activité ITP par rapport à **I-1.** Par ailleurs, le remplacement du noyau quinazoline par d'autres hétérocycles a permis d'indentifier des molécules montrant une capacité à inhiber la polymérisation de la tubuline à des concentrations sub-micromolaires.

### REFERENCES

1. G.R. Pettit et al J. Nat. Prod. 1987, 50, 119.
2. Mc Gown et al Cancer Chemother. Pharmacol. 1990, 26, 79.
3. G. G. Dark et al Cancer Res. 1997, 57, 1829.
4. (a) S. Messaoudi et al J. Med. Chem. 2009, 52, 4538. (b) M. Alami et al WO 2008/122620 (c) A. Hamze et al ChemMedChem. 2009, 4, 1912.
5. M. Soussi et al ChemMedChem, 2011, 6, 1781.
6. M. Alami et al WO 2009/147217.
7. Shelanski, M.C.; Gaskin, F.; Cantor, C.R. Proc. Natl. Acad. Sci. USA, 1973, 70, 765-768.
8. Gaskin, F.; Cantor, C.R.; Shelanski, M. C. J. Biol. Mol., 1974, 89, 737-755.
9. Shelanski, M. L.; Gaskin, F.; Cantor, C. R. Proc. Natl. Acad. Sci. USA, 1973, 70, 765-768.
10. Barron, D. M.; Chatterjee, S. K.; Ravindra, R.; Roof, R.; Baloglu, E.; Kingston, D. G. I.; Bane, S. Anal. Biochem. 2003, 315 49-56.

## Revendications

1. Composé de formule (Ib) suivante : 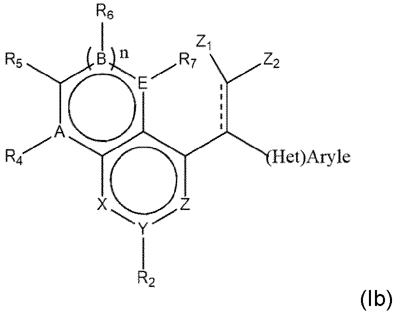 dans laquelle :
- A, B, E, Y et Z représentent un atome de carbone,
- n est égal à 1,
- X représente un atome d'azote ;
- R₄, R₅, R₆ et R₇ représentent, indépendamment l'un de l'autre :
- un atome d'hydrogène ;
- un atome d'halogène ;
- un groupe hydroxyle ;
- un groupe alkyle en C₁ à C₆ ;
- un groupe alcényle en C₂ à C₄ ;
- un groupe alcynyle en C₂ à C₄;
- un groupe alkoxy en C₁ à C₆ ; ou
- un groupe -NR'R" avec R' et R" représentant, indépendamment l'un de l'autre un hydrogène ou un groupe alkyle en C₁ à C₆ ;
- la liaison en pointillé est présente;
- R₂ représente :
- un atome d'hydrogène ;
- un atome d'halogène ;
- un groupe hydroxyle ;
- un groupe cyano ;
- un groupe -COYR' avec Y désignant 0 ou N et R' désignant H ou un groupe alkyle en C₁ à C₆, un groupe alcényle en C₂ à C₄, un groupe alcynyle en C₂ à C₄ ;
- un groupe -SO₂NR'R" avec R', R", désignant chacun, indépendamment l'un de l'autre, H ou un groupe alkyle en C₁ à C₆, un groupe alcényle en C₂ à C₄, un groupe alcynyle en C₂ à C₄ ;
- un groupe -NHSO₂R' avec R' désignant un groupe alkyle en C₁ à C₆, un groupe alcényle en C₂ à C₄, un groupe alcynyle en C₂ à C₄, un groupe aryle, un groupe hétéroaryle;
- un groupe alkyle en C₁ à C₆ ;
- un groupe alcényle en C₂ à C₄ ;
- un groupe alcynyle en C₂ à C₄;
- un groupe alkoxy en C₁ à C₆ ; ou
- un groupe -NR'R" avec R' et R" représentant, indépendamment l'un de l'autre un hydrogène ou un groupe alkyle ;
- Z₁ et Z₂ représentent indépendamment l'un de l'autre un atome d'hydrogène, un atome d'halogène ou un groupe méthyle ;
- (Het)Aryle représente un groupe aryle ou hétéroaryle, ledit hétéroaryle étant choisi parmi les groupes indolyle, benzothiophényle et benzofuranyle, ledit aryle ou hétéroaryle pouvant être substitué par un ou plusieurs substituants choisis parmi un atome d'halogène, un groupe hydroxyle, -OMe, -SMe, -OCX₃ avec X désignant un atome d'halogène, -NH₂, 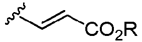 avec R désignant un groupe alkyle en C₁ à C₆, 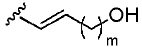 avec m représentant 1 ou 2, 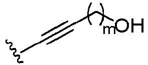 avec m représentant 1 ou 2 ou 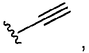
ainsi que ses sels pharmaceutiquement acceptables, ses stéréoisomères et ses prodrogues résultant de l'acylation ou de la phosphorylation d'un groupement hydroxy ou amino.

2. Composé selon l'une des revendications précédentes dans lequel (Het)Aryle désigne un groupe phényle pouvant être substitué par un ou plusieurs substituants choisis parmi un atome d'halogène, un groupe hydroxyle, -OMe, -SMe, -OCX₃ avec X désignant un atome d'halogène, - NH₂, 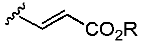 avec R désignant un groupe alkyle en C₁ à C₆, 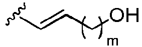 avec m représentant 1 ou 2, 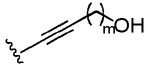 avec m représentant 1 ou 2 ou

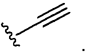

3. Composé selon la revendication 2 dans lequel le groupe phényle présente la formule suivante : 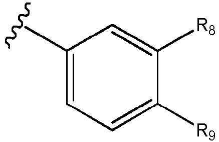 dans laquelle :
- R₈ représente un atome d'hydrogène, un atome d'halogène, un hydroxyle, -OMe, -SMe, -NH₂, 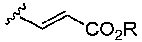 avec R désignant un groupe alkyle en C₁ à C₆, 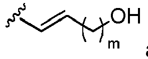 avec m représentant 1 ou 2, 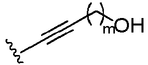 avec m représentant 1 ou 2 ou 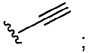 et
- Rₑ représente un groupement alkoxy en C₁, -SMe ou -OCX₃ avec X désignant un atome d'halogène.

4. Composé selon la revendication 1 présentant la formule (Ic) suivante 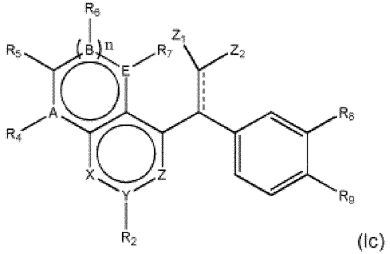 dans laquelle :
- n, A, B, E, X, Y, Z, R₄, R₅, R₆, R₇ sont tels que définis à la revendication 1;
- la liaison en pointillé est présente;
- R₂ représente :
- un atome d'hydrogène ;
- un atome d'halogène ;
- un groupe hydroxyle ;
- un groupe cyano ;
- un groupe -COYR' avec Y désignant O ou N et R' désignant H ou un groupe alkyle en C₁ à C₆, un groupe alcényle en C₂ à C₄, un groupe alcynyle en C₂ à C₄ ;
- un groupe -SO₂NR'R" avec R', R", désignant chacun, indépendamment l'un de l'autre, H ou un groupe alkyle en C₁ à C₆, un groupe alcényle en C₂ à C₄, un groupe alcynyle en C₂ à C₄ ;
- un groupe -NHSO₂R' avec R' désignant un groupe alkyle en C₁ à C₆, un groupe alcényle en C₂ à C₄, un groupe alcynyle en C₂ à C₄, un groupe aryle, un groupe hétéroaryle;
- un groupe alkyle en C₁ à C₆ ;
- un groupe alcényle en C₂ à C₄ ;
- un groupe alcynyle en C₂ à C₄;
- un groupe alkoxy en C₁ à C₆ ; ou
- un groupe -NR'R" avec R' et R" représentant, indépendamment l'un de l'autre un hydrogène ou un groupe alkyle ;
- Z₁ et Z₂ représentent indépendamment l'un de l'autre un atome d'hydrogène, un atome d'halogène ou un groupe méthyle ;
- R₈ représente un atome d'halogène, un hydroxyle, -OMe, -SMe, -NH₂, 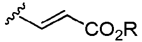 avec R désignant un groupe alkyle en C₁ à C₆, 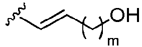 avec m représentant 1 ou 2, 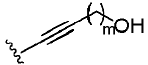 avec m représentant 1 ou 2 ou 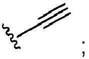 et
- R_{g} représente OMe, -SMe, -OCX₃ avec X désignant un atome d'halogène ;
ainsi que ses sels pharmaceutiquement acceptables, ses stéréoisomères et ses prodrogues résultant de l'acylation ou de la phosphorylation d'un groupement hydroxy ou amino.

5. Composé selon la revendication 1 présentant la formule suivante : 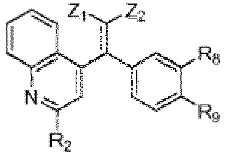 dans laquelle :
- Z₁, Z₂, et R₂ sont tels que définis à la revendication 1, R₈ et R₉ sont tels que définis à la revendication 3 ;
- la liaison en pointillé est présente.

6. Composé selon l'une des revendications précédentes, **caractérisé en ce que** R₉ représente un groupement -OMe, -SMe ou -OCX₃ avec X désignant un atome d'halogène.

7. Composé selon l'une des revendications précédentes, **caractérisé en ce que** R₈ représente un atome d'hydrogène, un atome d'halogène, un groupe hydroxyle ou un groupe - NH₂.

8. Composé selon l'une des revendications précédentes dans lequel Z₁ et Z₂ représentent un atome d'hydrogène ou dans lequel Z₁ et Z₂ représentent un atome de fluor.

9. Composé selon l'une des revendications précédentes représenté par la formule : 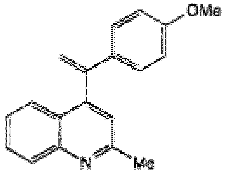 ainsi que ses sels pharmaceutiquement acceptables, ses stéréoisomères.

10. Composé, ainsi que ses sels pharmaceutiquement acceptables, ses stéréoisomères et ses prodrogues résultant de l'acylation ou de la phosphorylation d'un groupement hydroxy ou amino, selon l'une quelconque des revendications 1 à 9 pour son utilisation en tant que médicament.

11. Conjugué comprenant un composé selon l'une quelconque des revendications 1 à 9 lié de manière covalente à un anticorps.

12. Composition pharmaceutique comprenant au moins un composé selon l'une quelconque des revendications 1 à 9 ou un conjugué selon la revendication 11.

13. Composition pharmaceutique comprenant :
(i) au moins un composé selon l'une quelconque des revendications 1 à 9 et
(ii) au moins un autre principe actif,
en tant que produit de combinaison pour une utilisation simultanée, séparée ou étalée dans le temps.

14. Procédé de préparation d'un composé selon l'une quelconque des revendications 1 à 9 comprenant les étapes successives suivantes :
a) réaction d'un composé de formule (II) 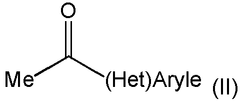 avec la tosylhydrazine pour conduire au composé tosylhydrazone de formule (III) 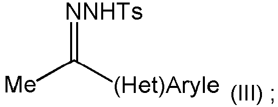
b) couplage du composé de formule (III) avec le composé de formule (V) 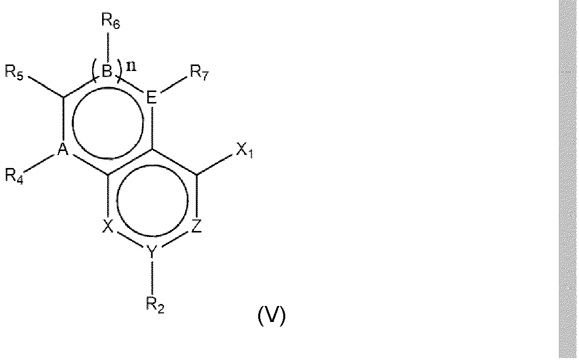 dans laquelle R₂, R₄, R₅, R₆, R₇, X, Y, Z, A, B, E et n sont tels que définis à l'une quelconque des revendications 1 à 9 et X₁ représente un atome d'halogène ou trifluorométhanesulfonate ;
c) séparation du milieu réactionnel du composé selon l'une quelconque des revendications 1 à 9 obtenu suite à l'étape b).

## Patentansprüche

1. Verbindung der folgenden Formel (Ib): 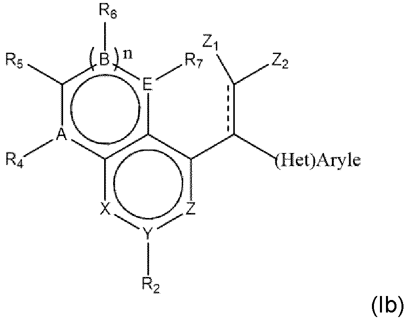 Wobei:
- A, B, E, Y und Z stehen für ein Kohlenstoffatom,
- n ist gleich 1,
- X stellt ein Stickstoffatom dar;
- R₄, R₅, R₆ und R₇ stehen unabhängig voneinander für :
- ein Wasserstoffatom ;
- ein Halogenatom ;
- eine Hydroxylgruppe ;
- eine Alkylgruppe mit C₁ bis C₆;
- eine Alkenylgruppe mit C₂ bis C₄;
- eine Alkynylgruppe mit C₂ bis C₄;
- eine Alkoxygruppe mit C₁ bis C₆; oder
- eine Gruppe -NR'R", wobei R' und R" unabhängig voneinander Wasserstoff oder eine Alkylgruppe mit C₁ bis C₆ darstellen;
- die gestrichelte Bindung ist vorhanden;
- R₂ steht für:
- ein Wasserstoffatom ;
- ein Halogenatom ;
- eine Hydroxylgruppe ;
- eine Cyanogruppe ;
- eine -COYR'-Gruppe, wobei Y für O oder N steht und R' für H oder eine Alkylgruppe mit C₁ bis C₆, eine Alkenylgruppe mit C₂ bis C₄, eine Alkinylgruppe mit C₂ bis C₄ steht;
- eine Gruppe -SO₂NR'R", wobei R' und R" jeweils unabhängig voneinander H oder eine Alkylgruppe mit C₁ bis C₆, eine Alkenylgruppe C₂ bis C₄, eine Alkinylgruppe C₂ bis C₄ bezeichnen;
- eine Gruppe -NHSO₂R', wobei R' eine Alkylgruppe mit C₁ bis C₆, eine Alkenylgruppe mit C₂ bis C₄, eine Alkinylgruppe mit C₂ bis C₄, eine Arylgruppe, eine Heteroarylgruppe bezeichnet;
- eine Alkylgruppe mit C₁ bis C₆;
- eine Alkenylgruppe mit C₂ bis C₄;
- eine Alkynylgruppe mit C₂ bis C₄;
- eine Alkoxygruppe mit C₁ bis C₆; oder
- eine -NR'R"-Gruppe, wobei R' und R" unabhängig voneinander ein Wasserstoffatom oder eine Alkylgruppe darstellen;
- Z₁ und Z₂ stellen unabhängig voneinander ein Wasserstoffatom, ein Halogenatom oder eine Methylgruppe dar;
- (Het)Aryl stellt eine Aryl- oder Heteroarylgruppe dar, wobei die Heteroarylgruppe aus den Indolyl-, Benzothiophenyl- und Benzofuranylgruppen ausgewählt ist, wobei die Aryl- oder Heteroarylgruppe durch einen oder mehrere Substituenten substituiert sein kann, die aus einem Halogenatom, einer Hydroxylgruppe, -OMe, -SMe, -OCX₃, wobei X für ein Halogenatom steht, -NH₂, 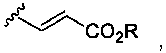 wobei R für eine Alkylgruppe mit C₁ bis C₆ steht, 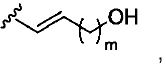 wobei m für 1 oder 2 steht, 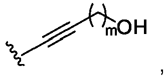 wobei m für 1 oder 2 steht, oder 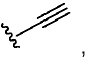 sowie deren pharmazeutisch annehmbare Salze, deren Stereoisomere und deren Prodrugs, die durch Acylierung oder Phosphorylierung einer Hydroxy- oder Aminogruppe entstehen.

2. Verbindung nach einem der vorhergehenden Ansprüche, worin (Het)Aryl eine Phenylgruppe bezeichnet, die durch einen oder mehrere Substituenten, ausgewählt aus einem Halogenatom, einer Hydroxylgruppe, -OMe, -SMe, -OCX₃, wobei X ein Halogenatom bezeichnet, - NH₂, 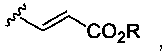 wobei R eine Alkylgruppe mit C₁ bis C₆ bezeichnet, 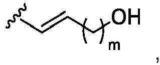 wobei m 1 oder 2 darstellt, 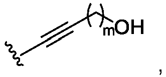 wobei m 1 oder 2 darstellt, oder 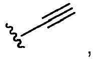 substituiert sein kann.

3. Verbindung nach Anspruch 2, wobei die Phenylgruppe die folgende Formel hat: 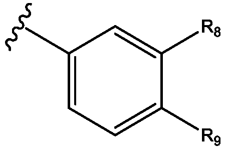 wobei :
- Rₐ ein Wasserstoffatom, ein Halogenatom, eine Hydroxylgruppe, -OMe, -SMe, -NH₂, 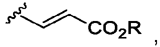 wobei R eine Alkylgruppe mit C₁ bis C₆ bezeichnet, 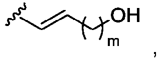 wobei m 1 oder 2 darstellt, 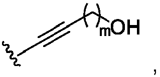 wobei m 1 oder 2 darstellt, oder 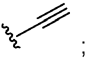 und
- R_{g} eine Alkoxygruppe mit C₁, -SMe oder -OCX₃ darstellt, wobei X ein Halogenatom bezeichnet.

4. Verbindung nach Anspruch 1, die die folgende Formel (Ic) aufweist 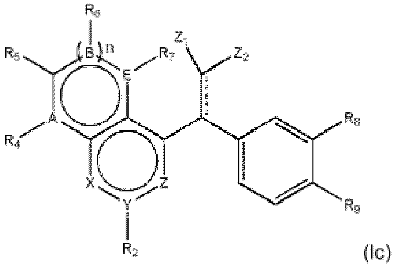 wobei:
- n, A, B, E, X, Y, Z, R₄, R₅, R₆, R₇ sind wie in Anspruch 1 definiert;
- die gestrichelte Bindung ist vorhanden;
- R₂ steht für:
- ein Wasserstoffatom ;
- ein Halogenatom ;
- eine Hydroxylgruppe ;
- eine Cyanogruppe ;
- eine -COYR'-Gruppe, wobei Y für O oder N steht und R' für H oder eine Alkylgruppe mit C₁ bis C₆, eine Alkenylgruppe mit C₂ bis C₄ oder eine Alkinylgruppe mit C₂ bis C₄ steht;
- eine Gruppe -SO₂NR'R", wobei R' und R" jeweils unabhängig voneinander H oder eine Alkylgruppe mit C₁ bis C₆, eine Alkenylgruppe mit C₂ bis C₄, eine Alkinylgruppe mit C₂ bis C₄ darstellen;
- eine Gruppe -NHSO₂R', wobei R' eine Alkylgruppe mit C₁ bis C₆, eine Alkenylgruppe mit C₂ bis C₄, eine Alkinylgruppe mit C₂ bis C(₄), eine Arylgruppe, eine Heteroarylgruppe bezeichnet;
- eine Alkylgruppe mit C₁ bis C₆;
- eine Alkenylgruppe mit C₂ bis C₄;
- eine Alkynylgruppe mit C₂ bis C₄;
- eine Alkoxygruppe mit C₁ bis C₆; oder
- eine -NR'R"-Gruppe, wobei R' und R" unabhängig voneinander ein Wasserstoffatom oder eine Alkylgruppe darstellen;
- Z₁ und Z₂ stellen unabhängig voneinander ein Wasserstoffatom, ein Halogenatom oder eine Methylgruppe dar;
- R₈ ein Halogenatom, ein Hydroxyl, -OMe, -SMe, -NH₂, 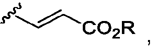 wobei R eine Alkylgruppe mit C₁ bis C₆ bezeichnet, 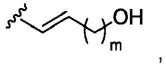 wobei m 1 oder 2 darstellt, 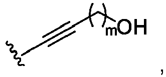 wobei m 1 oder 2 darstellt, oder 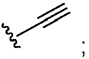 und
- R₉ bedeutet OMe, -SMe, -OCX₃, wobei X ein Halogenatom bezeichnet;
sowie deren pharmazeutisch annehmbare Salze, deren Stereoisomere und deren Prodrugs, die durch Acylierung oder Phosphorylierung einer Hydroxy- oder Aminogruppe entstehen.

5. Verbindung nach Anspruch 1, die die folgende Formel aufweist: 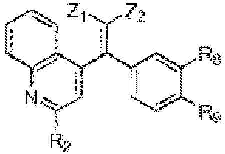 wobei:
- Z₁, Z₂ und R₂ sind wie in Anspruch 1 definiert, R₈ und R₉ sind wie in Anspruch 3 definiert
- die gepunktete Grenze ist vorhanden.

6. Verbindung nach einem der vorhergehenden Ansprüche, **dadurch gekennzeichnet, daß** R₉ eine Gruppe -OMe, -SMe oder -OCX₃ darstellt, wobei X ein Halogenatom bezeichnet.

7. Verbindung nach einem der vorhergehenden Ansprüche, **dadurch gekennzeichnet, daß** R₈ ein Wasserstoffatom, ein Halogenatom, eine Hydroxylgruppe oder eine Gruppe -NH₂ darstellt.

8. Verbindung nach einem der vorhergehenden Ansprüche, worin Z₁ und Z₂ ein Wasserstoffatom darstellen oder worin Z₁ und Z₂ ein Fluoratom darstellen.

9. Verbindung nach einem der vorangehenden Ansprüche, dargestellt durch die Formel: 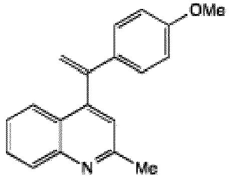 sowie ihre pharmazeutisch annehmbaren Salze und ihre Stereoisomere.

10. Verbindung sowie die pharmazeutisch akzeptablen Salze davon, die Stereoisomere davon und die Prodrugs davon, die aus der Acylierung oder Phosphorylierung einer Hydroxy- oder Aminogruppe resultieren, nach einem der Ansprüche 1 bis 9 für ihre Verwendung als Arzneimittel.

11. Konjugat, umfassend eine Verbindung nach einem der Ansprüche 1 bis 9, die kovalent an einen Antikörper gebunden ist.

12. Pharmazeutische Zusammensetzung, umfassend mindestens eine Verbindung nach einem der Ansprüche 1 bis 9 oder ein Konjugat nach Anspruch 11.

13. Pharmazeutische Zusammensetzung enthaltend:
(i) mindestens eine Verbindung nach einem der Ansprüche 1 bis 9 und
(ii) mindestens einen weiteren Wirkstoff,
als Kombinationsprodukt zur gleichzeitigen, getrennten oder gestaffelten Verwendung.

14. Verfahren zur Herstellung einer Verbindung nach einem der Ansprüche 1 bis 9, umfassend die folgenden aufeinanderfolgenden Schritte:
a) Reaktion einer Verbindung der Formel (II) 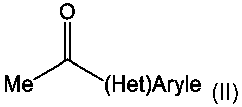 mit Tosylhydrazin, um die Tosylhydrazonverbindung der Formel (III) zu erhalten 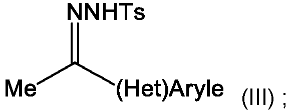
b) Kopplung der Verbindung der Formel (III) mit der Verbindung der Formel (V) 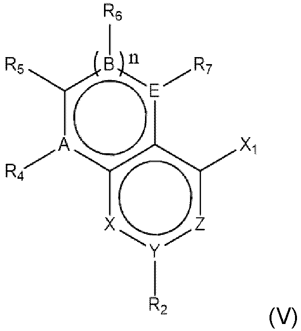 in der R₂, R₄, R₅, R₆, R₇, X, Y, Z, A, B, E und n die in einem der Ansprüche 1 bis 9 angegebenen Bedeutungen besitzen und X₁ ein Halogenatom oder Trifluormethansulfonat darstellt;
c) Abtrennung des Reaktionsmediums von der Verbindung nach einem der Ansprüche 1 bis 9, die als Ergebnis von Schritt b) erhalten wurde.

## Claims

1. Compound of the following formula (Ib) : 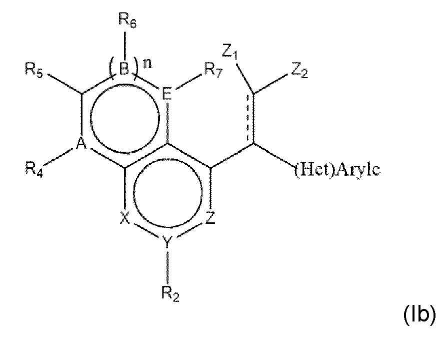 wherein :
- A, B, E, Y and Z represent a carbon atom,
- n is equal to 1,
- X represents a nitrogen atom;
- R₄, R₅, R₆ and R₇ represent, independently of each other :
- a hydrogen atom ;
- a halogen atom ;
- a hydroxyl group ;
- an alkyl group with C₁ to C₆;
- an alkenyl group with C₂ to C₄ ;
- an alkynyl group with C₂ to C₄;
- an alkoxy group with C₁ to C₆; or
- an -NR'R" group with R' and R" representing, independently of each other, a hydrogen or an alkyl group with C₁ to C₆;
- the dotted bond is present;
- R₂ represents :
- a hydrogen atom ;
- a halogen atom ;
- a hydroxyl group ;
- a cyano group ;
- a -COYR' group with Y designating O or N and R' designating H or an alkyl group with C₁ to C₆, an alkenyl group with C₂ to C₄, an alkynyl group with C₂ to C₄;
- an -SO₂NR'R" group where R' and R", each designating , independently of each other, H or an alkyl group with C₁ to C₆, an alkenyl group C₂ to C₄, an alkynyl group C₂ to C₄;
- an -NHSO₂R' group with R' designating an alkyl group with C₁ to C₆, an alkenyl group with C₂ to C₄, an alkynyl group with C₂ to C₄, an aryl group, a heteroaryl group;
- an alkyl group with C₁ to C₆;
- an alkenyl group with C₂ to C₄;
- an alkynyl group with C₂ to C₄;
- an alkoxy group with C₁ to C₆; or
- an -NR'R" group with R' and R" representing, independently of each other, a hydrogen or an alkyl group;
- Z₁ and Z₂ represent, independently of each other, a hydrogen atom, a halogen atom or a methyl group;
- (Het)Aryl represents an aryl or heteroaryl group, said heteroaryl being chosen from the indolyl, benzothiophenyl and benzofuranyl groups, said aryl or heteroaryl may be substituted by one or more substituents chosen from a halogen atom, a hydroxyl group, -OMe, -SMe, -OCX₃ with X designating a halogen atom, -NH₂, 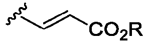 with R designating an alkyl group with C₁ to C₆, 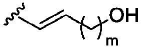 with m designating 1 or 2, 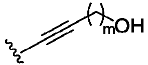 with m designating 1 or 2 or 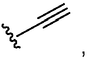
as well as the pharmaceutically acceptable salts thereof, the stereoisomers thereof and the prodrugs thereof resulting from the acylation or phosphorylation of a hydroxy or amino group.

2. Compound according to one of the preceding claims wherein (Het)Aryl designates a phenyl group which may be substituted by one or more substituents chosen from a halogen atom, a hydroxyl group, -OMe, -SMe, -OCX₃ with X designating a halogen atom, - NH₂, 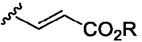 with R designating an alkyl group with C₁ to C₆, 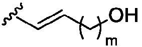 with m representing 1 or 2, 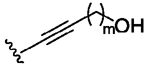 with m representing 1 or 2 or

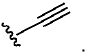

3. Compound according to claim 2 wherein the phenyl group has the following formula : 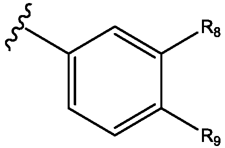 wherein :
- R₈ represents a hydrogen atom, a halogen atom, a hydroxyl, -OMe, -SMe, -NH₂, 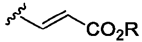 with R designating an alkyl group with C₁ to C₆, 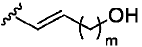 with m representing 1 or 2, 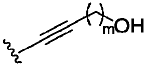 with m representing 1 or 2 or 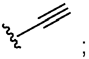 and
- R_{g} represents an alkoxy group with C₁, -SMe or -OCX₃, with X designating a halogen atom.

4. Compound according to claim 1, having the following formula (Ic) 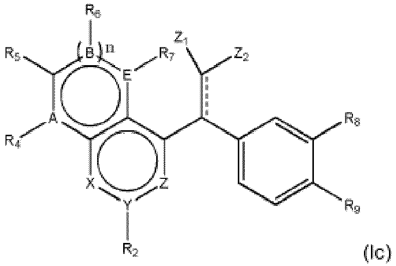 wherein :
- n, A, B, E, X, Y, Z, R₄, R₅, R₆, R₇ are as defined in claim 1;
- the dotted bond is present;
- R₂ represents :
- a hydrogen atom ;
- a halogen atom;
- a hydroxyl group ;
- a cyano group ;
- a -COYR' group with Y designating O or N and R' designating H or an alkyl group with C₁ to C₆, an alkenyl group C₂ to C₄ or an alkynyl group with C₂ to C₄;
- an -SO₂NR'R" group with R' and R", each representing, independently of each other, H or an alkyl group with C₁ to C₆, an alkenyl group with C₂ to C₄, an alkynyl group with C₂ to C₄;
- an -NHSO₂R' group with R' designating an alkyl group with C₁ to C₆, an alkenyl group with C₂ to C₄, an alkynyl group with C₂ to C₄, an aryl group, a heteroaryl group;
- an alkyl group with C₁ to C₆;
- an alkenyl group with C₂ to C₄;
- an alkynyl group with C₂ to C₄;
- an alkoxy group with C₁ to C₆; or
- an -NR'R" group with R' and R" representing, independently of each other, a hydrogen or an alkyl group;
- Z₁ and Z₂ represent independently of each other a hydrogen atom, a halogen atom or a methyl group;
- R₈ represents a halogen atom, a hydroxyl, -OMe, -SMe, -NH₂, 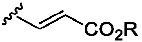 with R designating an alkyl group with C₁ to C₆, 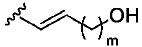 with m representing 1 or 2, 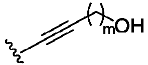 with m representing 1 or 2 or 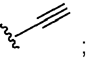 and
- R₉ represents OMe, -SMe, -OCX₃ with X designating a halogen atom;
as well as the pharmaceutically acceptable salts thereof, the stereoisomers thereof and the prodrugs thereof resulting from the acylation or phosphorylation of a hydroxy or amino group.

5. Compound according to claim 1, having the following formula : 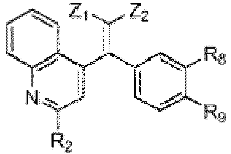 wherein :
- Z₁, Z₂, and R₂ are as defined in claim 1, R₈ and R₉ are as defined in claim 3
- the dotted bound is present.

6. Compound according to one of the preceding claims, **characterized in that** R_{g} represents an -OMe, -SMe or -OCX₃ group with X designating a halogen atom.

7. Compound according to one of the preceding claims, **characterized in that** R₈ represents a hydrogen atom, a halogen atom, a hydroxyl group or an -NH₂ group.

8. Compound according to one of the preceding claims wherein Z₁ and Z₂ represent a hydrogen atom or wherein Z₁ and Z₂ represent a fluorine atom.

9. A compound according to one of the preceding claims represented by the formula : 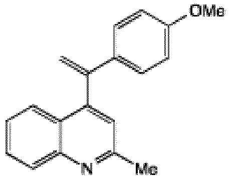 as well as the pharmaceutically acceptable salts thereof, the stereoisomers thereof.

10. Compound, as well as the pharmaceutically acceptable salts thereof, the stereoisomers thereof and the prodrugs thereof resulting from the acylation or phosphorylation of a hydroxy or amino group, according to any one of claims 1 to 9 for its use as a medicine.

11. Conjugate comprising a compound according to any one of claims 1 to 9 covalently bonded to an antibody.

12. A pharmaceutical composition comprising at least one compound according to any one of claims 1 to 9 or a conjugate according to claim 11.

13. Pharmaceutical composition comprising :
(i) at least one compound according to any one of claims 1 to 9 and
(ii) at least one other active ingredient,
as a combination product for simultaneous, separate or staggered use.

14. Method for preparing a compound according to any one of claims 1 to 9 comprising the following successive steps:
a) reaction of a compound of formula (II) 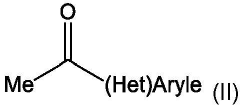 with tosylhydrazine to give the tosylhydrazone compound of formula (III) 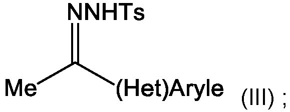
b) coupling of the compound of formula (III) with the compound of formula (V) 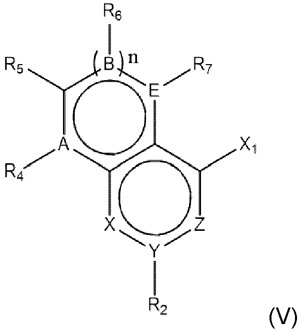 in which R₂, R₄, R₅, R₆, R₇, X, Y, Z, A, B, E and n are as defined in any one of claims 1 to 9 and X₁ represents a halogen atom or trifluoromethanesulfonate;
c) separation of the reaction medium from the compound according to any one of claims 1 to 9 obtained as a result of step b).
